# EUROPEAN PATENT APPLICATION

(11) **EP 4 386 002 A1**
(43) Date of publication of application: **19.06.2024**
(21) Application number: 22855485.3
(22) Date of filing: 11.08.2022
(51) Int. Cl.: C07K 16/24, C12N 15/13, C12N 15/85, C12N 5/10, A61K 39/395, A61P 1/16, A61P 11/00, A61P 9/04, G01N 33/68

(54) **IL-11 HUMANIZED ANTIBODY AND APPLICATION THEREOF**

(30) Priority: 12.08.2021 CN 202110924318
(71) Applicant: Sunshine Lake Pharma Co., Ltd., Dongguan, Guangdong 523000 (CN)
(72) Inventor: ZHOU, Jiatao, Dongguan, Guangdong 523871 (CN); CHEN, Cangsha, Dongguan, Guangdong 523871 (CN); WANG, Yincai, Dongguan, Guangdong 523871 (CN); JIE, Miaoxing, Dongguan, Guangdong 523871 (CN); DONG, Junji, Dongguan, Guangdong 523871 (CN); LI, Wenjia, Dongguan, Guangdong 523871 (CN)
(74) Representative: Hoffmann Eitle
(86) International application number: PCT/CN2022/111608
(87) International publication number: WO 2023/016505

(57) **Abstract**

Provided is an antibody or an antigen-binding fragment thereof capable of specifically recognizing IL-11. The antibody comprises heavy chain variable region CDRs as shown in SEQ ID NOs: 4-6, light chain variable region CDRs as shown in SEQ ID NOs: 37-39, heavy chain framework regions as shown in SEQ ID NOs: 67-70, and light chain framework regions as shown in SEQ ID NOs: 71-74, respectively. The antibody has low immunogenicity and can specifically target and bind to IL-11, so as to block binding of IL-11 to an IL-11 receptor.

## Description

### FIELD OF THE INVENTION

The present invention relates to the field of biotechnology. Specifically, the present invention relates to an IL-11 antibody and use thereof. More specifically, the present invention relates to an antibody or an antigen-binding fragment thereof that can specifically recognize IL-11, a nucleic acid molecule, an expression vector, a recombinant cell, a pharmaceutical composition, pharmaceutical use, a kit for detecting IL-11, and use for preparing detection or diagnostic kit.

### BACKGROUND ART

Fibrosis is an important process and a critical part of wound healing. Excessive fibrosis is common in many rare and common diseases and is important in disease pathogenesis. Diseases characterized by excessive fibrosis include, but are not limited to: systemic sclerosis, scleroderma, hypertrophic cardiomyopathy, dilated cardiomyopathy (DCM), atrial fibrillation, ventricular fibrillation, myocarditis, cirrhosis, kidney disease, eye disease, asthma, cystic fibrosis, arthritis and idiopathic pulmonary fibrosis. Despite the great impact on human health, there is still an unmet medical need for treatments and diagnostic methods for fibrosis.

The true physiological role of interleukin 11 (IL-11) is unclear. IL-11 is most closely related to hematopoietic cell activation and thrombopoiesis, but has also been found to have pro- and anti-inflammatory, pro-angiogenic effects, and is important for neoplasia. TGFβ1 or tissue damage is known to induce the expression of IL-11. From the published literature, the role of IL-11 in fibrosis is unclear. IL-11 is thought to be important for pulmonary fibrosis and inflammation, and its expression levels correlate with collagen levels in the skin and respiratory system. However, most studies indicate that IL-11 is anti-fibrotic in the heart and kidney, and anti-inflammatory in several tissues and chronic inflammatory diseases. In general, the molecular mode of action of IL-11 is thought to regulate the mRNA level of RNA expression through STAT3-mediated transcription.

The agents capable of inhibiting the action of IL-11 can prevent or reduce the binding of IL-11 to the IL-11 receptor. Studies have demonstrated the administration of agents capable of inhibiting the action of interleukin 11 (IL-11) can treat, prevent or alleviate fibrosis in subjects in need of treatment. Therefore, research on antibodies that can effectively bind IL-11 is of great value for the treatment of fibrotic diseases.

### SUMMARY

The present invention develops a humanized anti-IL-11 antibody with high neutralizing activity in order to treat or prevent fibrosis-related diseases.

In the first aspect of the present invention, the present invention provides an antibody or an antigen-binding fragment thereof that can specifically recognize IL-11. According to the embodiments of the present invention, the antibody comprises CDRs selected from at least one of the following or an amino acid sequence having at least 95% identity thereto: a heavy chain variable region comprising CDRs having the amino acid sequence of SEQ ID NO: 1 ~ 33, a light chain variable region comprising CDRs having the amino acid sequence of SEQ ID NO: 34 ~ 66, the antibody or antigen-binding fragment thereof has humanization modifications. It should be noted that the "humanization modification" described in this application refers to amino acid changes that can reduce the immunogenicity of the antibody or antigen-binding fragment thereof, including the mutation, insertion, deletion of amino acid, duplication of chemical group, and so on.
DYTFTNYW (SEQ ID NO: 4).
IYPGGGYT (SEQ ID NO: 5).
ARVRSGNDALDF (SEQ ID NO: 6).
ESVDEYGISF (SEQ ID NO: 37).
SAS (SEQ ID NO: 38).
QQSKEVPWT (SEQ ID NO: 39).

According to the embodiments of the present invention, the immunogenicity of the above-mentioned humanized antibody is reduced, and the antibody can specifically target and bind IL-11, and block the binding of IL-11 and IL-11 receptor.

According to the embodiments of the present invention, the above-mentioned antibody or antigen-binding fragment may further include at least one of the following additional technical features:

According to the embodiments of the present invention, the antibody comprises: a heavy chain variable region comprising CDR1, CDR2, CDR3 as shown in SEQ ID NO: 4, 5 and 6 or an amino acid sequence having at least 95% identity to SEQ ID NO: 4, 5 and 6, respectively.

According to the embodiments of the present invention, the antibody comprises: a light chain variable region comprising CDR1, CDR2, CDR3 as shown in SEQ ID NO: 37, 38 and 39 or an amino acid sequence having at least 95% identity to SEQ ID NO: 37, 38 and 39, respectively.

According to the embodiments of the present invention, the antibody or antigen-binding fragment thereof specifically recognizes IL-11.

According to the embodiments of the present invention, the antibody comprises at least one of a heavy chain framework region sequence and a light chain framework region sequence, and at least a part of at least one of the heavy chain framework region sequence and the light chain framework region sequence is derived from at least one of a murine antibody, a human antibody, a primate antibody, or a mutant thereof.

According to the embodiments of the present invention, the heavy chain framework region comprises FL1, FL2, FL3 and FL4, and the FL1, FL2, FL3 and FL4 of the heavy chain framework region having an amino acid sequence shown in SEQ ID NO: 67, 68, 69 and 70, respectively,
QVQLVQSGAEVKKPGASVKVSCKAS (SEQ ID NO: 67),
LGWVRQAPGX1GLEWX2GH (SEQ ID NO: 68),
NYNEKFKGRX3TX4TXSDTSTSTVYMELSSLRSEDTAVYX6C (SEQ ID NO: 69),
WGQGTLVTVSS (SEQ ID NO: 70),
wherein, X1 is Q or H, X2 is M or I, X3 is V or A, X4 is M or L, X5 is R or A, and X6 is Y or F.

According to the embodiments of the present invention, the light chain framework region comprises FL1, FL2, FL3 and FL4, and the FL1, FL2, FL3 and FL4 of the light chain framework region having an amino acid sequence shown in SEQ ID NO: 71, 72, 73 and 74, respectively,
EIVLTQSPATLSLSPGERATLSCRAS (SEQ ID NO: 71),
MNWX7QQKPGQAPRLLIY (SEQ ID NO: 72),
NQGSGIPARFSGSGSGTDFTLTISSLEPEDFAX8YX9C (SEQ ID NO: 73),
FGGGTKLEIK (SEQ ID NO: 74),
wherein, X7 is Y or F, X8 is V or M, and X9 is Y or F.

According to the embodiments of the present invention, the antibody has a heavy chain variable region with an amino acid sequence shown in any one of SEQ ID NO: 75 ~ 78.

QVQLVQSGAEVKKPGASVKVSCKASDYTFTNYWLGWVRQAPGQGLEWMGH
IYPGGGYTNYNEKFKGRVTMTRDTSTSTVYMELSSLRSEDTAVYYCARVRSGNDALDFWG
QGTLVTVSS (SEQ ID NO: 75).

QVQLVQSGAEVKKPGASVKVSCKASDYTFTNYWLGWVRQAPGQGLEWIGHI
YPGGGYTNYNEKFKGRVTMTADTSTSTVYMELSSLRSEDTAVYFCARVRSGNDALDFWGQ
GTLVTVSS (SEQ ID NO: 76).

QVQLVQSGAEVKKPGASVKVSCKASDYTFTNYWLGWVRQAPGQGLEWIGHI
YPGGGYTNYNEKFKGRATLTADTSTSTVYMELSSLRSEDTAVYFCARVRSGNDALDFWGQ
GTLVTVSS (SEQ ID NO: 77).

QVQLVQSGAEVKKPGASVKVSCKASDYTFTNYWLGWVRQAPGHGLEWIGHI
YPGGGYTNYNEKFKGRATLTADTSTSTVYMELSSLRSEDTAVYFCARVRSGNDALDFWGQ
GTLVTVSS (SEQ ID NO: 78).

According to the embodiments of the present invention, the antibody has a light chain variable region with an amino acid sequence shown in any one of SEQ ID NO: 79 ~ 82.

EIVLTQSPATLSLSPGERATLSCRASESVDEYGISFMNWYQQKPGQAPRLLIYSA
SNQGSGIPARFSGSGSGTDFTLTISSLEPEDFAVYYCQQSKEVPWTFGGGTKLEIK (SEQ ID
NO: 79).

EIVLTQSPATLSLSPGERATLSCRASESVDEYGISFMNWYQQKPGQAPRLLIYSA
SNQGSGIPARFSGSGSGTDFTLTISSLEPEDFAVYFCQQSKEVPWTFGGGTKLEIK (SEQ ID
NO: 80).

EIVLTQSPATLSLSPGERATLSCRASESVDEYGISFMNWFQQKPGQAPRLLIYSA
SNQGSGIPARFSGSGSGTDFTLTISSLEPEDFAVYFCQQSKEVPWTFGGGTKLEIK (SEQ ID
NO: 81).

EIVLTQSPATLSLSPGERATLSCRASESVDEYGISFMNWFQQKPGQAPRLLIYSA
SNQGSGIPARFSGSGSGTDFTLTISSLEPEDFAMYFCQQSKEVPWTFGGGTKLEIK (SEQ ID
NO: 82).

According to the embodiments of the present invention, the antibody comprises at least one of a heavy chain constant region and a light chain constant region, and at least a part of at least one of the heavy chain constant region and the light chain constant region is derived from at least one of a murine antibody, a human antibody, a primate antibody, or a mutant thereof.

According to the embodiments of the present invention, the full-length sequence of the antibody constant region is shown in SEQ ID NO: 83 or 84.






 (SEQ ID NO: 83).


 (SEQ ID
NO: 84).

Wherein, the full-length sequence of the antibody constant region shown in the above SEQ ID NO: 83 includes an IgG1 heavy chain constant region and a Fc region, wherein the sequence of the IgG1 heavy chain constant region is ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKV, the sequence of the Fc region is EPKSCDKTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTIS KAKGQPREPQVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK; the full-length sequence of the antibody constant region shown in the above SEQ ID NO: 84 is an IgG light chain constant region.

According to the embodiments of the present invention, the antibody has a heavy chain with an amino acid sequence shown in any one of SEQ ID NO: 85 ~ 88 and a light chain with an amino acid sequence shown in any one of SEQ ID NO: 89 ~ 92.

QVQLVQSGAEVKKPGASVKVSCKASDYTFTNYWLGWVRQAPGQGLEWMGH
IYPGGGYTNYNEKFKGRVTMTRDTSTSTVYMELSSLRSEDTAVYYCARVRSGNDALDFWG
QGTLVTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDKSRW

QQGNVFSCSVMHEALHNHYTQKSLSLSPGK (SEQ ID NO: 85).

QVQLVQSGAEVKKPGASVKVSCKASDYTFTNYWLGWVRQAPGQGLEWIGHI
YPGGGYTNYNEKFKGRVTMTADTSTSTVYMELSSLRSEDTAVYFCARVRSGNDALDFWGQ
GTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDKSRW
QQGNVFSCSVMHEALHNHYTQKSLSLSPGK (SEQ ID NO: 86).

QVQLVQSGAEVKKPGASVKVSCKASDYTFTNYWLGWVRQAPGQGLEWIGHI
YPGGGYTNYNEKFKGRATLTADTSTSTVYMELSSLRSEDTAVYFCARVRSGNDALDFWGQ
GTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDKSRW
QQGNVFSCSVMHEALHNHYTQKSLSLSPGK (SEQ ID NO: 87).

QVQLVQSGAEVKKPGASVKVSCKASDYTFTNYWLGWVRQAPGHGLEWIGHI
YPGGGYTNYNEKFKGRATLTADTSTSTVYMELSSLRSEDTAVYFCARVRSGNDALDFWGQ
GTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDKSRW
QQGNVFSCSVMHEALHNHYTQKSLSLSPGK (SEQ ID NO: 88).

EIVLTQSPATLSLSPGERATLSCRASESVDEYGISFMNWYQQKPGQAPRLLIYSA
SNQGSGIPARFSGSGSGTDFTLTISSLEPEDFAVYYCQQSKEVPWTFGGGTKLEIKRTVAAPS
VFIFPPSDEQLKSGTASVVCLLNNFYPREAKVQWKVDNALQSGNSQESVTEQDSKDSTYSL
SSTLTLSKADYEKHKVYACEVTHQGLSSPVTKSFNRGECC (SEQ ID NO: 89).

EIVLTQSPATLSLSPGERATLSCRASESVDEYGISFMNWYQQKPGQAPRLLIYSA
SNQGSGIPARFSGSGSGTDFTLTISSLEPEDFAVYFCQQSKEVPWTFGGGTKLEIKRTVAAPSV

FIFPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC (SEQ ID NO: 90).

EIVLTQSPATLSLSPGERATLSCRASESVDEYGISFMNWFQQKPGQAPRLLIYSA
SNQGSGIPARFSGSGSGTDFTLTISSLEPEDFAVYFCQQSKEVPWTFGGGTKLEIKRTVAAPSV
FIFPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC (SEQ ID NO: 91).

EIVLTQSPATLSLSPGERATLSCRASESVDEYGISFMNWFQQKPGQAPRLLIYSA
SNQGSGIPARFSGSGSGTDFTLTISSLEPEDFAMYFCQQSKEVPWTFGGGTKLEIKRTVAAPS
VFIFPPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC (SEQ ID NO: 92).

According to the embodiments of the present invention, the antibody is a single-chain antibody, a multimeric antibody, a CDR-grafted antibody or a small molecule antibody.

According to the embodiments of the present invention, the small molecule antibody comprises at least one of Fab antibody, Fv antibody, single domain antibody and minimal recognition unit.

In the second aspect of the present invention, the present invention provides a nucleic acid molecule. According to the embodiments of the present invention, the nucleic acid molecule encodes the aforementioned antibody or antigen-binding fragment thereof. The antibody or antigen-binding fragment encoded by the nucleic acid molecule according to the embodiments of the present invention has low immunogenicity and can specifically target and bind to IL-11, and block the binding of IL-11 and IL-11 receptor.

According to the embodiments of the present invention, the above-mentioned nucleic acid molecule may further include at least one of the following additional technical features:

According to the embodiments of the invention, the nucleic acid molecule is DNA.

According to the embodiments of the present invention, the nucleic acid molecule has a nucleotide sequence shown in any one of SEQ ID NO: 93 ~ 96 or has an nucleotide sequence shown in any one of SEQ ID NO: 97 ~ 100.

CAAGTGCAGCTGGTTCAGAGCGGAGCTGAGGTGAAGAAGCCTGGAGCTAG
CGTGAAGGTGTCTTGTAAAGCCAGCGACTACACCTTTACCAATTACTGGCTGGGCTGGG
TCAGACAGGCCCCTGGCCAGGGCCTGGAATGGATGGGCCACATCTACCCCGGCGGAGG
CTACACAAACTACAACGAGAAGTTCAAGGGCAGAGTGACAATGACCAGAGATACAAGC

ACCAGCACAGTGTATATGGAACTGTCTAGCCTGCGGAGCGAGGACACCGCCGTGTACTA
CTGCGCCAGAGTGCGGTCCGGCAACGACGCCCTGGATTTCTGGGGCCAGGGCACCCTG
GTGACCGTGTCCAGCGCCAGCACCAAGGGCCCTTCCGTGTTTCCACTGGCCCCCTCCTC
TAAATCCACATCTGGCGGCACCGCCGCCCTGGGCTGTCTGGTGAAGGACTACTTCCCAG
AGCCTGTGACAGTGTCCTGGAACTCTGGCGCCCTGACATCCGGCGTGCACACATTTCCA
GCCGTGCTGCAGAGCTCCGGCCTGTACAGCCTGTCTAGCGTGGTGACAGTGCCCTCCTC
TAGCCTGGGCACACAGACCTATATCTGCAACGTGAATCACAAGCCAAGCAATACCAAGG
TGGACAAGAAGGTGGAGCCCAAGTCCTGTGATAAGACACACACCTGCCCCCCTTGTCCT
GCTCCCGAGCTGCTGGGCGGCCCTAGCGTGTTCCTGTTTCCACCCAAGCCTAAGGACAC
CCTGATGATCTCCCGGACACCCGAGGTGACCTGCGTGGTGGTGGACGTGTCTCACGAGG
ATCCTGAGGTGAAGTTCAACTGGTATGTGGATGGCGTGGAGGTGCACAATGCCAAGACC
AAGCCCAGAGAGGAGCAGTACAACTCTACATATAGGGTGGTGAGCGTGCTGACCGTGCT
GCACCAGGACTGGCTGAACGGCAAGGAGTATAAGTGCAAGGTGTCCAATAAGGCCCTG
CCCGCCCCCATCGAGAAGACAATCAGCAAGGCCAAGGGCCAGCCTCGGGAGCCACAGG
TGTACACCCTGCCTCCATCCAGAGACGAGCTGACAAAGAACCAGGTGTCTCTGACATGT
CTGGTGAAGGGCTTCTATCCTAGCGATATCGCCGTGGAGTGGGAGTCCAATGGCCAGCC
AGAGAACAATTACAAGACCACACCCCCTGTGCTGGACTCCGATGGCTCCTTCTTTCTGTA
TTCCAAGCTGACCGTGGATAAGTCTCGGTGGCAGCAGGGCAACGTGTTCAGCTGTTCCG
TGATGCACGAAGCCCTGCATAATCACTATACTCAGAAATCCCTGTCCCTGTCACCTGGAA
AGTGATAA (SEQ ID NO: 93).

CAAGTGCAGCTGGTCCAGAGCGGCGCCGAGGTGAAAAAGCCTGGAGCTAG
CGTGAAGGTGTCCTGCAAGGCCAGCGACTACACCTTCACCAACTATTGGCTGGGCTGGG
TGCGGCAGGCCCCTGGACAGGGCCTGGAATGGATCGGCCACATCTACCCCGGAGGCGG
CTACACCAATTACAACGAGAAGTTCAAGGGCAGAGTGACCATGACCGCCGATACAAGC
ACATCTACAGTGTACATGGAACTGAGCAGCCTGAGAAGCGAGGATACAGCTGTTTACTT
CTGTGCCAGAGTGCGGTCCGGCAACGACGCCCTGGACTTTTGGGGCCAGGGCACCCTG
GTGACCGTGTCTAGCGCCAGCACCAAGGGCCCTTCCGTGTTTCCACTGGCCCCCTCCTC
TAAATCCACATCTGGCGGCACCGCCGCCCTGGGCTGTCTGGTGAAGGACTACTTCCCAG
AGCCTGTGACAGTGTCCTGGAACTCTGGCGCCCTGACATCCGGCGTGCACACATTTCCA
GCCGTGCTGCAGAGCTCCGGCCTGTACAGCCTGTCTAGCGTGGTGACAGTGCCCTCCTC
TAGCCTGGGCACACAGACCTATATCTGCAACGTGAATCACAAGCCAAGCAATACCAAGG

TGGACAAGAAGGTGGAGCCCAAGTCCTGTGATAAGACACACACCTGCCCCCCTTGTCCT
GCTCCCGAGCTGCTGGGCGGCCCTAGCGTGTTCCTGTTTCCACCCAAGCCTAAGGACAC
CCTGATGATCTCCCGGACACCCGAGGTGACCTGCGTGGTGGTGGACGTGTCTCACGAGG
ATCCTGAGGTGAAGTTCAACTGGTATGTGGATGGCGTGGAGGTGCACAATGCCAAGACC
AAGCCCAGAGAGGAGCAGTACAACTCTACATATAGGGTGGTGAGCGTGCTGACCGTGCT
GCACCAGGACTGGCTGAACGGCAAGGAGTATAAGTGCAAGGTGTCCAATAAGGCCCTG
CCCGCCCCCATCGAGAAGACAATCAGCAAGGCCAAGGGCCAGCCTCGGGAGCCACAGG
TGTACACCCTGCCTCCATCCAGAGACGAGCTGACAAAGAACCAGGTGTCTCTGACATGT
CTGGTGAAGGGCTTCTATCCTAGCGATATCGCCGTGGAGTGGGAGTCCAATGGCCAGCC
AGAGAACAATTACAAGACCACACCCCCTGTGCTGGACTCCGATGGCTCCTTCTTTCTGTA
TTCCAAGCTGACCGTGGATAAGTCTCGGTGGCAGCAGGGCAACGTGTTCAGCTGTTCCG
TGATGCACGAAGCCCTGCATAATCACTATACTCAGAAATCCCTGTCCCTGTCACCTGGAA
AGTGATAA (SEQ ID NO: 94).

CAAGTGCAGCTGGTCCAGAGCGGCGCCGAGGTGAAAAAGCCTGGCGCTTC
TGTGAAGGTGTCCTGCAAGGCCAGCGACTACACATTTACCAATTATTGGCTGGGCTGGG
TGCGGCAGGCCCCTGGACAGGGCCTGGAATGGATCGGCCACATCTACCCCGGCGGAGG
CTACACAAACTACAACGAGAAGTTCAAGGGCAGAGCCACACTCACCGCTGATACCAGC
ACAAGCACCGTGTACATGGAACTGAGCAGCCTGAGAAGCGAGGACACCGCCGTGTACT
TCTGTGCCAGAGTGCGGTCCGGCAACGACGCCCTGGATTTCTGGGGACAGGGCACCCT
GGTGACCGTGTCTAGCGCCAGCACCAAGGGCCCTTCCGTGTTTCCACTGGCCCCCTCCT
CTAAATCCACATCTGGCGGCACCGCCGCCCTGGGCTGTCTGGTGAAGGACTACTTCCCA
GAGCCTGTGACAGTGTCCTGGAACTCTGGCGCCCTGACATCCGGCGTGCACACATTTCC
AGCCGTGCTGCAGAGCTCCGGCCTGTACAGCCTGTCTAGCGTGGTGACAGTGCCCTCCT
CTAGCCTGGGCACACAGACCTATATCTGCAACGTGAATCACAAGCCAAGCAATACCAAG
GTGGACAAGAAGGTGGAGCCCAAGTCCTGTGATAAGACACACACCTGCCCCCCTTGTC
CTGCTCCCGAGCTGCTGGGCGGCCCTAGCGTGTTCCTGTTTCCACCCAAGCCTAAGGAC
ACCCTGATGATCTCCCGGACACCCGAGGTGACCTGCGTGGTGGTGGACGTGTCTCACGA
GGATCCTGAGGTGAAGTTCAACTGGTATGTGGATGGCGTGGAGGTGCACAATGCCAAGA
CCAAGCCCAGAGAGGAGCAGTACAACTCTACATATAGGGTGGTGAGCGTGCTGACCGT
GCTGCACCAGGACTGGCTGAACGGCAAGGAGTATAAGTGCAAGGTGTCCAATAAGGCC
CTGCCCGCCCCCATCGAGAAGACAATCAGCAAGGCCAAGGGCCAGCCTCGGGAGCCAC

AGGTGTACACCCTGCCTCCATCCAGAGACGAGCTGACAAAGAACCAGGTGTCTCTGAC
ATGTCTGGTGAAGGGCTTCTATCCTAGCGATATCGCCGTGGAGTGGGAGTCCAATGGCCA
GCCAGAGAACAATTACAAGACCACACCCCCTGTGCTGGACTCCGATGGCTCCTTCTTTC
TGTATTCCAAGCTGACCGTGGATAAGTCTCGGTGGCAGCAGGGCAACGTGTTCAGCTGT
TCCGTGATGCACGAAGCCCTGCATAATCACTATACTCAGAAATCCCTGTCCCTGTCACCT
GGAAAGTGATAA (SEQ ID NO: 95).

CAAGTGCAGCTGGTTCAGAGCGGCGCCGAGGTGAAGAAGCCTGGCGCTAG
CGTGAAGGTGTCTTGTAAAGCCAGCGACTACACATTTACCAATTACTGGCTGGGATGGG
TCCGGCAGGCCCCTGGCCACGGCCTGGAATGGATCGGCCACATCTACCCCGGCGGAGGA
TATACAAACTACAACGAGAAGTTCAAGGGCAGAGCCACCCTGACCGCTGATACATCTAC
AAGCACCGTGTACATGGAACTGAGCAGCCTGAGAAGCGAGGATACCGCCGTGTACTTCT
GCGCCAGAGTGCGGTCCGGCAACGACGCCCTGGACTTCTGGGGCCAGGGCACCCTGGT
GACCGTGTCCAGCGCCAGCACCAAGGGCCCTTCCGTGTTTCCACTGGCCCCCTCCTCTA
AATCCACATCTGGCGGCACCGCCGCCCTGGGCTGTCTGGTGAAGGACTACTTCCCAGAG
CCTGTGACAGTGTCCTGGAACTCTGGCGCCCTGACATCCGGCGTGCACACATTTCCAGC
CGTGCTGCAGAGCTCCGGCCTGTACAGCCTGTCTAGCGTGGTGACAGTGCCCTCCTCTA
GCCTGGGCACACAGACCTATATCTGCAACGTGAATCACAAGCCAAGCAATACCAAGGTG
GACAAGAAGGTGGAGCCCAAGTCCTGTGATAAGACACACACCTGCCCCCCTTGTCCTG
CTCCCGAGCTGCTGGGCGGCCCTAGCGTGTTCCTGTTTCCACCCAAGCCTAAGGACACC
CTGATGATCTCCCGGACACCCGAGGTGACCTGCGTGGTGGTGGACGTGTCTCACGAGGA
TCCTGAGGTGAAGTTCAACTGGTATGTGGATGGCGTGGAGGTGCACAATGCCAAGACCA
AGCCCAGAGAGGAGCAGTACAACTCTACATATAGGGTGGTGAGCGTGCTGACCGTGCTG
CACCAGGACTGGCTGAACGGCAAGGAGTATAAGTGCAAGGTGTCCAATAAGGCCCTGC
CCGCCCCCATCGAGAAGACAATCAGCAAGGCCAAGGGCCAGCCTCGGGAGCCACAGGT
GTACACCCTGCCTCCATCCAGAGACGAGCTGACAAAGAACCAGGTGTCTCTGACATGTC
TGGTGAAGGGCTTCTATCCTAGCGATATCGCCGTGGAGTGGGAGTCCAATGGCCAGCCA
GAGAACAATTACAAGACCACACCCCCTGTGCTGGACTCCGATGGCTCCTTCTTTCTGTAT
TCCAAGCTGACCGTGGATAAGTCTCGGTGGCAGCAGGGCAACGTGTTCAGCTGTTCCGT
GATGCACGAAGCCCTGCATAATCACTATACTCAGAAATCCCTGTCCCTGTCACCTGGAAA
GTGATAA (SEQ ID NO: 96).

GAGATCGTGCTGACACAGAGCCCTGCTACACTCTCCCTGTCCCCAGGCGAG

CGGGCCACACTGTCTTGTAGAGCCAGCGAGAGCGTCGACGAGTACGGCATTAGCTTCAT
GAACTGGTATCAGCAGAAACCTGGCCAGGCCCCTAGACTGCTGATCTACAGCGCCTCCA
ACCAGGGCAGCGGCATCCCCGCTAGATTCAGCGGCAGCGGATCTGGCACCGACTTCACC
CTGACCATCAGCAGCCTGGAACCCGAGGATTTTGCCGTGTACTACTGCCAGCAATCTAA
GGAAGTGCCTTGGACCTTCGGCGGAGGCACCAAGCTGGAAATCAAGAGGACAGTGGCC
GCCCCAAGCGTGTTCATCTTTCCCCCTTCCGACGAGCAGCTGAAGTCTGGCACCGCCAG
CGTGGTGTGCCTGCTGAACAACTTCTACCCTCGGGAGGCCAAGGTCCAGTGGAAGGTG
GATAACGCCCTGCAGTCTGGCAATAGCCAGGAGTCCGTGACCGAGCAGGACTCTAAGGA
TAGCACATATTCCCTGTCTAGCACCCTGACACTGAGCAAGGCCGATTACGAGAAGCACA
AGGTGTATGCCTGTGAAGTCACCCATCAGGGGCTGTCATCACCCGTCACTAAGTCATTCA
ATCGCGGAGAATGCTGATAA (SEQ ID NO: 97).

GAGATCGTGCTGACCCAGAGCCCCGCCACCCTGTCCCTGAGCCCTGGCGAG
CGGGCTACACTGTCTTGTAGAGCCAGCGAGAGCGTCGACGAGTACGGCATCTCTTTTAT
GAACTGGTATCAGCAGAAACCTGGACAGGCCCCTAGACTGCTGATCTACAGCGCCTCCA
ACCAGGGCAGCGGCATTCCTGCTAGATTCAGCGGCAGCGGCTCCGGCACAGATTTCACC
CTCACCATCAGCAGCCTGGAACCCGAGGACTTCGCCGTGTACTTCTGCCAGCAATCTAA
GGAAGTGCCATGGACATTCGGCGGAGGCACCAAGCTGGAAATCAAGAGGACAGTGGCC
GCCCCAAGCGTGTTCATCTTTCCCCCTTCCGACGAGCAGCTGAAGTCTGGCACCGCCAG
CGTGGTGTGCCTGCTGAACAACTTCTACCCTCGGGAGGCCAAGGTCCAGTGGAAGGTG
GATAACGCCCTGCAGTCTGGCAATAGCCAGGAGTCCGTGACCGAGCAGGACTCTAAGGA
TAGCACATATTCCCTGTCTAGCACCCTGACACTGAGCAAGGCCGATTACGAGAAGCACA
AGGTGTATGCCTGTGAAGTCACCCATCAGGGGCTGTCATCACCCGTCACTAAGTCATTCA
ATCGCGGAGAATGCTGATAA (SEQ ID NO: 98).

GAGATCGTGCTGACCCAGTCTCCAGCCACCCTCTCCCTGAGCCCCGGCGAG
CGGGCCACACTGTCTTGTAGAGCTAGCGAGAGCGTCGACGAGTACGGCATCTCCTTCAT
GAACTGGTTCCAGCAGAAACCTGGCCAGGCCCCTAGACTGCTGATCTACAGCGCCAGC
AACCAGGGCTCCGGAATTCCTGCTAGATTCAGCGGCTCTGGCAGCGGCACAGATTTTAC
CCTGACAATCAGCAGCCTGGAACCCGAGGACTTTGCCGTGTACTTCTGCCAGCAAAGCA
AGGAAGTGCCTTGGACCTTCGGCGGAGGCACCAAGCTGGAAATCAAGAGGACAGTGG
CCGCCCCAAGCGTGTTCATCTTTCCCCCTTCCGACGAGCAGCTGAAGTCTGGCACCGCC
AGCGTGGTGTGCCTGCTGAACAACTTCTACCCTCGGGAGGCCAAGGTCCAGTGGAAGG

TGGATAACGCCCTGCAGTCTGGCAATAGCCAGGAGTCCGTGACCGAGCAGGACTCTAAG
GATAGCACATATTCCCTGTCTAGCACCCTGACACTGAGCAAGGCCGATTACGAGAAGCA
CAAGGTGTATGCCTGTGAAGTCACCCATCAGGGGCTGTCATCACCCGTCACTAAGTCATT
CAATCGCGGAGAATGCTGATAA (SEQ ID NO: 99).

GAGATCGTGCTGACACAGAGCCCTGCTACACTCTCCCTGAGCCCCGGCGAG
CGGGCCACCCTGTCTTGTAGAGCCAGCGAGAGCGTGGACGAGTACGGCATTAGCTTCAT
GAACTGGTTTCAGCAGAAACCTGGCCAGGCCCCTAGACTGCTGATCTACAGCGCCTCCA
ACCAGGGCTCTGGCATCCCCGCTAGATTCAGCGGATCTGGCTCCGGCACCGACTTCACC
CTGACCATCAGCAGCCTGGAACCAGAGGATTTTGCCATGTACTTCTGCCAGCAAAGCAA
GGAAGTGCCTTGGACCTTCGGCGGAGGCACAAAGCTGGAAATCAAGAGGACAGTGGC
CGCCCCAAGCGTGTTCATCTTTCCCCCTTCCGACGAGCAGCTGAAGTCTGGCACCGCCA
GCGTGGTGTGCCTGCTGAACAACTTCTACCCTCGGGAGGCCAAGGTCCAGTGGAAGGT
GGATAACGCCCTGCAGTCTGGCAATAGCCAGGAGTCCGTGACCGAGCAGGACTCTAAG
GATAGCACATATTCCCTGTCTAGCACCCTGACACTGAGCAAGGCCGATTACGAGAAGCA
CAAGGTGTATGCCTGTGAAGTCACCCATCAGGGGCTGTCATCACCCGTCACTAAGTCATT
CAATCGCGGAGAATGCTGATAA (SEQ ID NO: 100).

In the third aspect of the present invention, the present invention provides an expression vector. According to the embodiments of the present invention, the expression vector carries the aforementioned nucleic acid molecule. After the expression vector according to the embodiments of the present invention is introduced into a suitable recipient cell, the expression of the aforementioned antibody or antigen-binding fragment thereof that specifically recognizes IL-11 can be effectively realized under the mediation of the regulatory system, thereby in vitro mass acquisition of the antibody or antigen-binding fragment are achieved.

According to the embodiments of the present invention, the above-mentioned expression vector may further include at least one of the following additional technical features:
According to the embodiments of the present invention, the expression vector is a eukaryotic expression vector. Thereby the aforementioned antibody or antigen-binding fragment thereof that specifically recognizes IL-11 can be expressed in eukaryotic cells, such as CHO cells.

In the fourth aspect of the present invention, the present invention provides a recombinant cell. According to the embodiments of the present invention, the recombinant cell carries the aforementioned nucleic acid molecules, or expresses the aforementioned antibody or antigen-binding fragment thereof. The recombinant cell according to the embodiments of the present invention can be used for in vitro expression and mass acquisition of the aforementioned antibody or antigen-binding fragment thereof that specifically recognizes IL-11.

According to the embodiments of the present invention, the above-mentioned recombinant cell may further include at least one of the following additional technical features:
According to the embodiments of the present invention, the recombinant cell is obtained by introducing the aforementioned expression vector into a host cell.

According to the embodiments of the present invention, the expression vector is introduced into the host cell by electrotransduction.

According to the embodiments of the present invention, the recombinant cell is a eukaryotic cell.

According to the embodiments of the present invention, the recombinant cell is a mammalian cell.

In a fifth aspect of the present invention, the present invention provides a pharmaceutical composition. According to the embodiments of the present invention, the pharmaceutical composition comprises the aforementioned antibody, the aforementioned nucleic acid molecule, the aforementioned expression vector or the aforementioned recombinant cell. The antibody contained or expressed in the pharmaceutical composition according to the embodiments of the present invention can specifically target and bind to IL-11, and block the binding of IL-11 to the IL-11 receptor.

In the sixth aspect of the present invention, the present invention provides the use of the aforementioned antibody, the aforementioned nucleic acid molecule, the aforementioned expression vector or the aforementioned recombinant cell, and the aforementioned pharmaceutical composition in the manufacture of a medicament, the medicament is used for treating pulmonary fibrosis, non-alcoholic steatohepatitis (NASH), chronic heart failure (CHF) or diseases caused by the transformation of fibroblasts into fibrosis.

In the seventh aspect of the present invention, the present invention provides a kit for detecting IL-11. According to the embodiments of the present invention, the kit includes the aforementioned antibodies. The aforementioned IL-11 antibody can specifically target and bind to IL-11. The kit according to the embodiments of the present invention can realize the specific detection of IL-11. For example, when the antibody is bound with a fluorescent group, a fluorescence detection can be used to achieve localization or real-time detection of IL-11.

In the eighth aspect of the present invention, the present invention provides use of the aforementioned antibody, the aforementioned nucleic acid molecule, the aforementioned expression vector or the aforementioned recombinant cell in the preparation of a kit, the kit is used to detect IL-11 or diagnose IL-11 related diseases.

Additional aspects and advantages of the present invention will be set forth in part form following description, and in part will be obvious from the following description, or may be learned by practice of the invention.

### Description of the drawings

The above and/or additional aspects and advantages of the present invention will become apparent and readily understood from the following description of the embodiments taken in conjunction with the following drawings, wherein:
Figure 1 is the result of competitive blocking of the binding of IL-11 and IL-11R by the preferred antibody according to the embodiments of the present invention;
Figure 2 is the detection result of competitive blocking activity of the preferred antibody according to the embodiments of the present invention;
Figure 3 is the western detection result of the preferred antibodies inhibiting the transformation of lung fibroblast HFL-1 to fibrosis according to the embodiment of the present invention;
Figure 4 is the statistical analysis result of the preferred antibodies inhibiting the transformation of lung fibroblast HFL-1 to fibrosis according to the embodiments of the present invention;
Figure 5 and 6 are results of inhibiting pulmonary fibrosis in C57BL/6 mice by the preferred antibody according to the embodiments of the present invention.
Figure 7 is a graph showing the thermal stability test results of the preferred humanized antibodies according to the embodiments of the present invention;
Figure 8 is the result of competitive blocking activity of the humanized antibodies according to the embodiments of the present invention;
Figure 9 is the lung inflammation damage score of SD rats treated with humanized antibodies according to the embodiments of the present invention;
Figure 10 is the pulmonary fibrosis score of SD rats treated with humanized antibodies according to the embodiments of the present invention;
Figure 11 is the result of affinity matured antibodies binding activity according to the embodiments of the present invention;
Figure 12 is the result of competitive blocking activity of the affinity matured antibodies according to the embodiments of the present invention; and
Figure 13 is the result of inhibiting the transformation of liver fibroblast LX-2 to fibrosis according to the embodiments of the present invention.

### EXAMPLES

The embodiments of the present invention are described in detail below, examples of the embodiments are illustrated in the accompanying drawings, wherein the same or similar labels throughout indicate the same or similar elements or elements with the same or similar functions.

Furthermore, the terms "first" and "second" are only used for descriptive purposes and should not be construed as indicating or implying relative importance or implying the number of indicated technical features. Thus, a feature delimited with "first" and "second" may explicitly or implicitly include at least one of that feature. In the description of the present invention, "plurality" means at least two, such as two, three, etc., unless otherwise expressly and specifically defined.

### Antibody

Herein, the term "antibody" includes intact antibody and any antigen-binding fragment (i.e., "antigen-binding part") or single chain thereof. Intact antibody is immunoglobulin molecule capable of binding to specific antigen. It consists of two light chains with lighter molecular weight and two heavy chains with heavier molecular weight. The heavy chain (H chain) and light chain (L chain) are connected by a disulfide bond to form a tetrapeptide chain molecule. Among them, the amino acid sequence of the amino-terminal (N-terminus) of the peptide chain varies greatly, which is called variable region (V region), and the carboxyl-terminal (C-terminus) is relatively stable and varies little, which is called constant region (C region). The V regions of the L chain and H chain are called VL and VH, respectively.

The term "antigen-binding fragment" (or "antibody fragment" for short) refers to an antibody fragment formed from a portion of an antibody comprising one or more CDRs. The antigen-binding function of the antibody has been shown to be performed by the fragments of full-length antibody. The examples of the antigen-binding part include, but are not limited to, the variable region, Fab, Fab", F (ab")₂, Fv fragment, disulfide bond-stabilized Fv Fragment (dsFv), (dsFv)₂, polyspecific antibody and nanobody. Furthermore, although the two domains VL and VH of the Fv fragment are encoded by separate genes, but they can be linked together using a recombinant method by a synthetic linker that enables them to form a protein chain, wherein the VL and VH regions pair to form a monovalent molecule (termed single- chain Fv (scFv)). This single-chain antibody is also expected to be included in the "antigen-binding fragment" of the term antibody. These antibody fragments are obtained using conventional techniques known to those skilled in the art, and the practicality of these fragments is screened using the same method as intact antibody. Antigen-binding fragment is able to bind the same antigen as the parental antibody. In some embodiments, an antigen-binding fragment may comprise one or more CDRs grafted onto a framework region from one or more different humanized antibodies.

The amino acid composition and arrangement sequence of certain regions in the variable region have a higher degree of variation, which is called hypervariable region (HVR), the hypervariable region is where the antigen and antibody bind, so it is also called complementarity-determining region (CDR). There are three CDR regions in both the heavy chain variable region and the light chain variable region. The amino acid composition and arrangement sequence outside the hypervariable region of the antibody variable region vary relatively little, which is called the framework region (FR). There are four framework regions in VH and VL, which are represented by FR1, FR2, FR3 and FR4, respectively.

The present invention uses an IL-11 antigen to obtain a highly specific and high-affinity anti-IL-11 Fab (antigen-binding fragment) antibody fragment through immunization. The antibody fragment can specifically bind to IL-11 antigen, so that diseases such as pulmonary fibrosis can be targeted for treatment.

It should be noted that "Immunogenicity" refers to the ability to induce an immune response, that is, the antigen can stimulate specific immune cells, to make the immune cells activate, proliferate, and differentiate, and finally produce the characteristics of immune effector antibodies and sensitized lymphocytes. The inventors of the present application further carried out humanization transformation (humanization modification) on the screened new neutralizing antibody sequences of murine origin to obtain a humanized antibody, which can not only retain the affinity and specificity of the parental mouse monoclonal antibody, but also greatly reduce its immunogenicity and improve its safety.

In some embodiments, the present invention provides a humanized antibody or antigen-binding fragment that can specifically recognize IL-11, the antibody comprises CDRs selected from at least one of the following or an amino acid sequence having at least 95% identity thereto: a heavy chain variable region comprising CDRs having an amino acid sequence of SEQ ID NO: 1 ~ 33, a light chain variable region comprising CDRs having an amino acid sequence of SEQ ID NO: 34 ~ 66, the antibody or antigen-binding fragment thereof has humanization modifications. In other embodiments, the antibody or antigen-binding fragment provided by the present invention has conservative amino acid substitution compared with the heavy chain and light chain mentioned above. "Antigen-binding fragment" refers to the antibody fragment that retains the ability to specifically bind an antigen. "Conservative amino acid substitution" refers to the replacement of an amino acid by another amino acid with a residue that is biologically, chemically, or structurally similar. Biologically similar means that the substitution does not destroy the biological activity of the IL-11 antibody or the binding to the IL-11 antigen. Structurally similar means that amino acids have side chains of similar length, such as alanine, glycine, or serine, or have side chains of similar size. The chemical similarity means that the amino acids have the same charge or are both hydrophilic or hydrophobic. For example, the hydrophobic residues isoleucine, valine, leucine or methionine are substituted for each other. Alternatively, polar amino acids can be substituted for each other, such as arginine substituted for lysine, glutamate substituted for aspartic acid, glutamine substituted for asparagine, serine substituted for threonine, etc.

In some embodiments, the present invention provides an antibody or antigen-binding fragment, which has a heavy chain variable region with an amino acid sequence shown in any one of SEQ ID NO: 75 ~ 78 and a light chain variable region with an amino acid sequence shown in any one of SEQ ID NO: 79 ~ 82. The inventors can obtain the CDR regions of the above-mentioned heavy chain variable region (as shown in SEQ ID NO: 4 ~ 6) and the CDR regions of the light chain variable region (as shown in SEQ ID NO: 37 ~ 39) through the antibody sequence alignment database (NCBI, IMGT). In other embodiments, the heavy chain variable region sequence of the antibody or antigen-binding fragment has conservative amino acid substitution compared to the amino acid sequence shown in SEQ ID NO: 75 ~ 78. In some embodiments, the light chain variable region sequence of the antibody or antigen-binding fragment has conservative amino acid substitution compared to the amino acid sequence shown in any one of SEQ ID NO: 79 ~ 82. Of course, these conservative amino acid substitutions do not alter the biological function of the antibody or antigen-binding fragment. In some embodiments, these conservative amino acid substitutions can occur at the amino acids in heavy chain variable regions and light chain variable regions other than CDR regions.

In some preferred embodiments, the present invention provides an anti-IL-11 antibody, which has a heavy chain with an amino acid sequence shown in any one of SEQ ID NO: 85 ~ 88 and a light chain with an amino acid sequence shown in any one of SEQ ID NO: 89 ~ 92.

In some preferred schemes, the present invention provides an anti-IL-11 single-chain antibody.

### Nucleic Acid Molecule, Expression Vector, Recombinant Cell

In the process of preparing or obtaining these antibodies, the nucleic acid molecules expressing these antibodies can be used to connect with different vectors, and then express in different cells to obtain corresponding antibodies.

To this end, the present invention also provides an isolated nucleic acid molecule, which encodes the antibody or antigen-binding fragment described above.

In some embodiments, the isolated nucleic acid molecule has a nucleotide sequence shown in any one of SEQ ID NO: 93 ~ 96 or has a nucleotide sequence shown in any one of SEQ ID NO: 97 ~ 100.

In some embodiments, the isolated nucleic acid molecule has at least more than 90% homology with the nucleotide sequences shown in the SEQ ID NO: 93 ~ 96 above, and preferably more than 95% homology, more preferably with 98%, 99% homology. In at least some embodiments, the isolated polynucleotide has at least more than 90% homology with the nucleotide sequence shown in the SEQ ID NO: 97 ~ 100, and preferably more than 95% homology, more preferably with more than 98%, 99% homology. These sequences have homology to the nucleotide sequences shown in SEQ ID NO: 93 ~ 96 or SEQ ID NO: 97 ~ 100, and can express amino acids similar to SEQ ID NO: 85 ~ 88 and SEQ ID NO: 89 ~ 92, thereby can specifically bind to the IL-11 antigen to achieve the targeting function of the antibody.

In some preferred embodiments, the isolated nucleic acid molecule has a heavy chain nucleotide sequence shown in SEQ ID NO: 93 ~ 96 and a light chain nucleotide sequence shown in SEQ ID NO: 97 ~ 100. These nucleotide sequences have been optimized for species and are more easily expressed in mammalian cells.

The present invention also provides an expression vector comprising the above-described isolated nucleic acid molecule. When the above-described isolated polynucleotide is ligated to a vector, the polynucleotide can be directly or indirectly connected to control elements on the vector, as long as these control elements can control the translation and expression and the like of the polynucleotide. Of course, these control elements can come directly from the vector itself, or can be exogenous, that is, not from the vector itself. Of course, the polynucleotide is operably linked to the control elements. Herein, "operably linked " refers to linking an exogenous gene to a vector, so that the control elements inside the vector, such as transcription control sequences and translation control sequences, etc., can perform the intended functions of regulating the transcription and translation of the exogenous gene. Of course, the polynucleotides used to encode the heavy and light chains of antibodies can be independently inserted into different vectors, and usually inserted into the same vector. Commonly used vectors can be, for example, plasmid, phage, and the like. For example, Plasmid-X plasmid.

The present invention also provides a recombinant cell comprising the expression vector. The expression vector can be introduced into mammalian cells to construct and obtain recombinant cells, and then these recombinant cells can be used to express the antibody or antigen-binding fragments provided by the present invention. By culturing the recombinant cells, corresponding antibodies can be obtained. These available mammalian cells can be, for example, CHO cells and the like.

### Pharmaceutical Composition, Kit and Pharmaceutical Use and Use in the Preparation of Kit.

The present invention also provides a pharmaceutical composition comprising the above-mentioned antibody or antigen-binding fragment and a pharmaceutically acceptable carrier.

The anti-IL-11 antibody presented herein can be incorporated into a pharmaceutical composition suitable for administration to a subject. Generally, these pharmaceutical compositions include the anti-IL-11 antibody provided herein and a pharmaceutically acceptable carrier.

"Pharmaceutically acceptable carrier" can include any and all solvents, dispersion media, coating, antibacterial and antifungal agents, isotonic agents, delayed absorbents, and the like that are physiologically compatible. Specific examples may be one or more of water, saline, phosphate buffered saline, glucose, glycerol, ethanol, and the like, and combinations thereof. In many cases, the pharmaceutical compositions include isotonic agents, such as sugars, polyols (such as mannitol, sorbitol), or sodium chloride, etc. Of course, the pharmaceutically acceptable carriers can also include trace amounts of auxiliary substances, such as wetting agents or emulsifiers, preservatives or buffering agents, to extend the shelf life or efficacy of antibody.

For example, the antibodies of the invention can be incorporated into pharmaceutical compositions suitable for parenteral administration (e.g., intravenous, subcutaneous, intraperitoneal, intramuscular). These pharmaceutical compositions can be prepared in various forms. Such as liquid, semisolid, and solid dosage forms, and the like, including, but not limited to, liquid solutions (e.g., injection solutions and infusion solutions), dispersions or suspensions, tablets, pills, powders, liposomes, and suppositories. Typical pharmaceutical compositions are in the form of injection solutions or infusion solutions. The antibody may be administered by intravenous infusion or injection or intramuscular or subcutaneous injection.

Of course, the anti-IL-11 antibody herein can be made into a kit or as part of other diagnostic reagents as needed. According to the embodiments of the present invention, the present invention also provides a kit comprising the IL-11 antibody described above. The application of the kit provided by the present invention, for example, the kit can be used for immunoblotting, immunoprecipitation, etc., involving the use of the specific binding properties of IL-11 antigen and antibody for detection, and the like. These kits may contain any one or more of the following: antagonist, anti-IL-11 antibody, or drug reference material; protein purification column; immunoglobulin affinity purification buffer; assay diluent for cells; instructions or literature, etc. Anti-IL-11 antibody can be used for different types of diagnostic tests, such as detection of a variety of diseases or the presence of drugs, toxins or other proteins in vitro or in vivo. For example, it can be used to test for related diseases by testing the serum or blood of the subject. Such related diseases may include IL-11 related diseases such as pulmonary fibrosis and the like. Of course, the antibody provided herein can also be used for radioimmunoassay and radioimmunotherapy of the above-described diseases.

When the anti-IL-11 antibody provided by the present invention is used to treat the above-mentioned diseases, the anti-IL-11 antibody provided herein can be provided to a subject. To this end, the present invention provides a method for treating and/or preventing the above-mentioned diseases, comprising administering the antibody or antigen-binding fragment thereof, nucleic acid molecule, expression vector, recombinant cell or pharmaceutical composition provided by the present invention to a subject in need.

The scheme of the present invention will be explained below in conjunction with the embodiments. Those skilled in the art will understand that the following examples are only used to illustrate the present invention, and should not be construed as limiting the scope of the present invention. If no specific technology or condition is specified in the embodiments, the technology or condition described in the literature in this field or the product specification shall be followed. The reagents or instruments used without the manufacturer's indication are conventional products that can be obtained from the market.

### Example 1 Production of Recombinant Human Protein Antigen Hu-IL-11-His Tag

Construction of vector: The amino acid sequence of human IL-11 was obtained from the global public database Uniprot (P20809:124294). A pcDNA3.4 expression vector (purchased from Thermo Fisher Scientific) containing human IL-11 gene sequence was constructed by molecular cloning method. The vector encoding human IL-11 was transfected into mammalian cells CHO by electrotransfection for expression for 14 days. After the expression was completed, the harvested cells supernatant was preliminarily purified by HisTrap HP (nickel column) (GE healthcare, Cat:17524801), and then Superdex75 (GE healthcare, Cat: 29148721) was used for refine purification, and finally a recombinant protein antigen hu-IL-11-His tag that can be used to immunize animals was obtained.

### Example 2 Animal Immunization

In order to obtain mice that can secrete antibody against hu-IL-11, the inventors immunized female Balb/c mice. Female Balb/c were purchased from Hunan SJA Laboratory Animal Co., Ltd. The recombinant protein antigen hu-IL-11-His tag obtained in Example 1 and the immune adjuvant were fully emulsified before animal immunization. The immunization process is shown in Table 1.

**Table 1: Schedule of Animal Immunization**

| Immunity time point (Day) | Immunogen dose | Immunization method |
|---|---|---|
| 0 | 50µg hu-IL-11-His tag and CFA mixed in equal volume | Intraperitoneal multi-point injection |
| 14 | 50µg hu-IL-11-His tag and IFA mixed in equal volume | Intraperitoneal multi-point injection |
| 28 | 50µg hu-IL-11-His tag and IFA mixed in equal volume | Intraperitoneal multi-point injection |
| 35 | None | Orbital blood collection |
| 38 | 50µg hu-IL-11-His tag | Tail vein injection |
| 41 | None | Mice were sacrificed and spleens were harvested |

A small amount of mice orbital blood was harvested on the 35th day, and the antibody titer was detected by conventional Elisa method.

### Example 3 Fusion and Screening of Antibody Secreting Cells

Isolated mouse splenocytes were fused with immortalized mouse myeloma cells SP2/0 and cultured in RPMI-1640 complete medium containing 10⁻⁴ M Hypoxanthine, 4×10⁻⁷ M Aminopterin, 1.6 ×10⁻⁵ M Thymidine for approximately 14 days, the antibody affinity was detected and screened after the fusion cells grew out the clone group. The antibody clones that bind to IL-11 were selected, the supernatants were taken, and the competitive binding of antigen and antibody was determined by flow cytometry. High secretion specific antibody cell lines were selected for expansion culture and cryopreservation, the results are shown in Table 2. The specific method was to obtain antibody clones with high affinity (OD450 nm was the determined affinity value) for IL-11 by Elisa screening firstly, wherein P.C. was the binding between the purchased IL-11 antibody (Abcam, ab130083) and IL-11 protein as positive control. Secondly, a cell line stably expressing IL-11R was constructed, and an appropriate amount of IL-11 protein was mixed with the hybridoma supernatant, and then co-incubated with a cell line stably expressing IL-11R, the blocking effect (mean value of FITC-A) of hybridoma supernatant on IL-11/IL-11R was finally detected by flow cytometry, wherein N.C. was the value detected without adding hybridoma supernatant, which could be regarded as the value without blocking at all (Negative control). The underline in Table 2 indicates the selected clones. After multiple rounds of subcloning, a monoclonal antibody derived from a single antibody-secreting cell was finally obtained. The affinity (OD450nm) and flow blocking results of the monoclonal antibodies detected are shown in Table 3.

**Table 2: Test results of Elisa affinity test and flow cytometry competition test**

| Clone(No.) | OD(450nm) value | FITC-A mean | Clone(No.) | OD(450nm) value | FITC-A mean | Clone(No.) | OD(450nm) value | FITC-A mean |
|---|---|---|---|---|---|---|---|---|
| 3-1A2 | 2.243 | 12538 | 3-12F10 | 0.628 | 17067 | 5-5F5 | 2.768 | 4564 |
| 3-2A12 | 1.781 | 13334 | 3-13F11 | 1.915 | 15967 | 5-5F8 | 2.532 | 9436 |
| 3-2B2 | 1.084 | 13285 | 3-13G12 | 2.732 | 15312 | 5-5F11 | 2.627 | 12273 |
| 3-2B5 | 3.432 | 13837 | 3-13H1 | 1.41 | 13551 | 5-6F1 | 2.677 | 11496 |
| 3-3B9 | 1.777 | 13049 | 3-13H8 | 2.066 | 14323 | 5-6G5 | 2.679 | 10201 |
| 3-3G9 | 3.078 | 11577 | 3-15A11 | 1.214 | 16661 | 5-7B4 | 2.751 | 6587 |
| 3-3G11 | 2.943 | 13959 | 3-15C4 | 1.318 | 15017 | 5-9E12 | 2.049 | 10707 |
| 3-4G12 | 2.991 | 14418 | 3-15D5 | 2.894 | 11919 | 5-9D7 | 2.129 | 10127 |
| 3-4H1 | 2.876 | 12658 | 3-15D10 | 2.402 | 15064 | 5-9G8 | 3.591 | 6747 |
| 3-4H4 | 2.736 | 14459 | 3-17D12 | 2.48 | 15572 | 5-8G1 | 3.714 | 10460 |
| 3-4H12 | 3.33 | 13876 | 3-17E7 | 2.372 | 13301 | 5-10D3 | 3.201 | 10041 |
| 3-5B2 | 3.264 | 11001 | 3-18C11 | 2.566 | 14884 | 5-10F6 | 2.311 | 10472 |
| 3-5D11 | 1.068 | 13000 | 3-18C12 | 3.504 | 16114 | 5-12B1 | 2.351 | 9782 |
| 3-5G12 | 1.907 | 14267 | 3-19E8 | 3.412 | 15279 | 5-14B10 | 2.54 | 11385 |
| 3-6A4 | 2.656 | 750 | 3-20F12 | 3.496 | 14504 | 5-14C3 | 3.654 | 7672 |
| 3-7C1 | 2.661 | 13349 | 5-1A2 | 2.765 | 6217 | 5-17C11 | 3.462 | 11718 |
| 3-7C6 | 2.589 | 11605 | 5-1B12 | 2.865 | 4613 | 5-20E11 | 3.649 | 4330 |
| 3-7C9 | 2.722 | 11406 | 5-1E9 | 2.531 | 4638 | 5-21G2 | 3.545 | 4946 |
| 3-7G12 | 2.959 | 12412 | 5-2E11 | 2.921 | 5218 | 5-21H7 | 3.25 | 11385 |
| 3-7H5 | 1.995 | 13161 | 5-2F11 | 2.275 | 5625 | 5-22C4 | 3.561 | 6019 |
| 3-8H12 | 2.926 | 13360 | 5-2G11 | 2.948 | 5045 | 5-24F6 | 3.571 | 9954 |
| 3-9C12 | 1.915 | 14499 | 5-3E8 | 2.775 | 4897 | 5-25D3 | 1.87 | 3713 |
| 3-9D12 | 2.732 | 13833 | 5-3G12 | 2.77 | 8573 | P.C.(Elisa) | 3.62 | N/A |
| 3-9E1 | 2.894 | 13822 | 5-4C9 | 2.034 | 5933 | N. C. (Flow) | N/A | 24596 |

| | | | | | | | | |
|---|---|---|---|---|---|---|---|---|
| Note: N/A in Table 2 means: no data | | | | | | | | |

**Table 3: Test results of Elisa affinity experiment and flow competition experiment**

| Sample number | OD450 | FITC Mean | Sample number | OD450 | FITC Mean |
|---|---|---|---|---|---|
| 3-6A4 | 1.584 | 3036 | 3-5B2-2G4 | 1.65 | 19751 |
| 5-20E11-5F2 | 1.596 | 3109 | 5-21G12-1D3 | 1.528 | 3362 |
| 5-20E11-1A2 | 1.634 | 3635 | 3-5B2-7A4 | 1.636 | 20958 |
| 3-15D5-3A6 | 1.688 | 10944 | 3-9E1-3A2 | 1.544 | 9067 |
| 3-7C9-1A6 | 1.601 | 3148 | 3-15D5-6A1 | 1.511 | 13639 |
| 3-2B5-3D2 | 1.614 | 15289 | N.C.(Flow) | N/A | 18433 |

| | | | | | |
|---|---|---|---|---|---|
| Note: N/A in Table 3 means: no data; because the sequence of 3-6A4 cells after subcloning was consistent with the sequence before subcloning, its number is the same as that in Table 2. | | | | | |

### Example 4 Sequence Determination of Monoclonal Antibody

1*10^6∼5*10^6 hybridoma cells were taken, the total RNA was extracted with Takara MiniBEST Universal RNA Extraction Kit, and cDNA was reverse transcribed with PrimeScript RT Reagent Kit (Takara Corporation). The above steps were performed according to the instructions provided by the manufacturer. The design of gene amplification primers for antibody heavy chain variable region (VH) and light chain variable region (VL) mainly refers to the primer pairs in Ig-Primer Sets (Novagen Corporation, Cat. No.: 69831-3), which were synthesized by BGI Genomics. The heavy chain variable region and light chain variable region genes were amplified from the first strand of the cDNA, then cloned into the pMD18-T vector and transformed into E. coli DH5α, the clones were selected and sequenced by BGI Genomics to obtain gene sequences of the heavy chain variable region (VH) and light chain variable region (VL). The antibody sequences obtained by sequencing were shown as SEQ ID NO: 101 ~ 122.

QIQLVQSGPELKKPGETVKISCKASGYTFTTNGINWVKQAAGKGLKWMGWIN
TNTGEPTNAEELKGRFAFSLETSASTAYLQINNLKNEDAAIYFCAREGAFGLDYWGQGTSV
TVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSRDELT
KNQVSLTCLVKGFYPSDIAVEWESNGQPENNYKTTPPVLDSDGSFFLYSKLTVDKSRWQQG
NVFSCSVMHEALHNHYTQKSLSLSPGK (SEQ ID NO: 101).

QVQLQQSGAELVRPGTSAKISCKASDYTFTNYWLGWLKQRPGHGLEWIGHIY
PGGGYTNYNEKFKGKATLTADTSSSTAYMQLSSLTSEDSAVYFCARVRSGNDALDFWGQGT
SVTVSSASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPELL
GGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLTVDKSRWQ
QGNVFSCSVMHEALHNHYTQKSLSLSPGK (SEQ ID NO: 102).

QIQLVQSGPELKKPGETVKISCKASGYTFTNYGMNWVKQAPGKGLKWMGWI
NTNTGEPTYAEEIKGRFAFSLETSASTAYLQINNLKNEDTATYFCSREGIYGMDSWGQGTSV
TVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSRDELT
KNQVSLTCLVKGFYPSDIAVEWESNGQPENNYKTTPPVLDSDGSFFLYSKLTVDKSRWQQG
NVFSCSVMHEALHNHYTQKSLSLSPGK (SEQ ID NO: 103).

QVQLKESGPGLVAPSQSLSITCTVSGFSLTSYGVHWVRQPPGKGLEWLGIIWAG
GRTDYNSALVSRLNISKDNSKSQVFLKMHSLQTDDTAMYYCAREGGYDYDGDLWTTGVK

EPQSPSPQASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDKSRW
QQGNVFSCSVMHEALHNHYTQKSLSLSPGK (SEQ ID NO: 104).

QVQLKQSGPGLVLPSQSLSITCTVSGFSLTSYGVHWFRQSPGTGLEWLGVIWS
GGITDYNATFI.PRLSISKDNSKSQVFFKMNSLQADDTAIYYCARNFDGYYYSIDYWGQGTS
VTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPELLG
GPSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLTVDKSRWQ
QGNVFSCSVMHEALHNHYTQKSLSLSPGK (SEQ ID NO: 105).

QVQLKQSGPGLVQPSQSLSITCTVSGFSLTNYGVHWVRQSPGKGLEWLGAIWR
GGSTDYNAAFMSRLSITKDNSKSQVFFKMNSLQPDDTAIYYCAKNLYGPAAMDYWGQGTS
VTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPELLG
GPSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLTVDKSRWQ
QGNVFSCSVMHEALHNHYTQKSLSLSPGK (SEQ ID NO: 106).

QIQLVQSGPELKKPGETVNISCRASGDTFTNYAVNWVKQAPGKGLKWMGWID
TYTGDPTYADDLKGRFAFSLETSATTAYLQINNLKNEDTATYFCAGDTWFAYWGQGTLVTV
SAASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSKLTVDKSRWQQGN
VFSCSVMHEALHNHYTQKSLSLSPGK (SEQ ID NO: 107).

QVTLKESGPGILQPSQTLSLTCSFSGFSLSSSGMSVGWIRQPSGKGLEWLAHIW

WSDDKSYNPALKSRLTISKDTSNNQVFLKIASVVTADTATYYCAREGSLGYGLDYWGQGT
TLTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPELLG
GPSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLTVDKSRWQ
QGNVFSCSVMHEALHNHYTQKSLSLSPGK (SEQ ID NO: 108).

EVKLLESGGGLVQPGGSLKLSCAASGFDFRRYWMSWVRQAPGKGLEWIGQIN
PDSSMINYTPSLKDKFIISRDNAKNTLYLQMSEVRSEDTALYYCATYGHHATDSWGQGTSV
TVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSRDELT
KNQVSLTCLVKGFYPSDIAVEWESNGQPENNYKTTPPVLDSDGSFFLYSKLTVDKSRWQQG
NVFSCSVMHEALHNHYTQKSLSLSPGK (SEQ ID NO: 109).

QVQLQQPGAELVRPGTSVKMSCKASGYTFTSYWMHWVKQRPGQGLEWIGDI
YPGSDSTNYHEKFKSKATLTVDTSSSTAYMQLSSLTSEDSAVYYCALDSSGYGFAYWGQGT
LVTVSAASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPELL
GGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLTVDKSRWQ
QGNVFSCSVMHEALHNHYTQKSLSLSPGK (SEQ ID NO: 110).

EVQLVESGGGLVQPGESLKLSCDSNDYEFPSHDMSWVRKTPEKRLELVAAINS
DGGNTYYPDTMERRFIISRDNTKRTLYLQMSSLRSEDTALHYCARPRPTIGTTATGSSMSGA
QGPRSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPELLG
GPSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLTVDKSRWQ
QGNVFSCSVMHEALHNHYTQKSLSLSPGK (SEQ ID NO: 111).

SIVMTQSPKFLLVSAGDRVIITCKASQSVSNDVVWYQQKPGQSPKLLIYYGSHR
NTGVPTRFTGSGYGTDFTFTISTVQAEDLAVYFCQQDFFSPWTFGGGTKLEIKRTVAAPSVFI
FPPSDEQLKSGTASVVCLLNNFYPREAKVQWKVDNALQSGNSQESVTEQDSKDSTYSLSST
LTLSKADYEKHKVYACEVTHQGLSSPVTKSFNRGEC (SEQ ID NO: 112).

DIVLTQSPASLAVSLGQRATISCRASESVDEYGISFMNWFQQKPGQPPRLLIYSA
SNQGSGVPARFTGSGSGTDFSLNILPMEEDDTAMYFCQQSKEVPWTFGGGTKLEIKRTVAA
PSVFIFPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC (SEQ ID NO: 113).

SIVMTQTPKFLLVSAGDRVTITCKASQSVSNDVVWYRQKPGQSPKLLIYYASN
RYIGVPDRFTGSGYGTDFTFTISTVQAEDLAVYFCQQDYYSPWTFGGGTKLEIKRTVAAPSV
FIFPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC (SEQ ID NO: 114).

DVVMTQSPLSLPVSLGDQASISCRSSQSLVHSNGNTYLHWYLQKPGQSPKLLI
YKVSNRFSGVPDRFSGSGSGTDFTLKISRVEAEDLGVYFCSQSTHVPPDVRWRHQAGNQRT
VAAPSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC (SEQ ID NO: 115).

DIQMTQTTSSLSASLGDRVTISCRASQDISNSLNWYQQKPDGTVKLLIYYTSRL
HSGVPSRFTGSGSGTDYSLTISNLEQEDIATYFCQQGNTLPLTFGAGTKLELKRTVAAPSVFIF
PPSDEQLKSGTASVVCLLNNFYPREAKVQWKVDNALQSGNSQESVTEQDSKDSTYSLSSTL
TLSKADYEKHKVYACEVTHQGLSSPVTKSFNRGEC (SEQ ID NO: 116).

DIQMNQSPSSLSASLGDTITITCHANQNIYVWLSWYQQKPGNIPKLLIYEASNL
HTGVPSRFSGSGSGTGFTLTISSLQPEDIATYYCQQGQSYPYTFGGGTKLEIKRTVAAPSVFIF
PPSDEQLKSGTASVVCLLNNFYPREAKVQWKVDNALQSGNSQESVTEQDSKDSTYSLSSTL
TLSKADYEKHKVYACEVTHQGLSSPVTKSFNRGEC (SEQ ID NO: 117).

DIVMSQSPSSLAVSVGEKVTMSCKSSQSLLYSRNQKNRLAWYQQKPGQSPKLL
IYWASTRESGVPDRFTGSGSGTDFTLTISSVKAEDLAVYYCQQYYSYPRTFGGGTNLEIKRTV
AAPSVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC (SEQ ID NO: 118).

DVLMTQTPLSLPVSLGDQASISCRSSQSIVHTDGNTYLEWYLQKPGQSPKLLIY
KVSNRFSGVPDRFSGSGSGTDFTLKISRVEAEDLGVYYCFQGSHVPWTFGGGTKLEIKRTVA
APSVFIFPPSDEQLKSGTASVVCLLNNFYPREAKVQWKVDNALQSGNSQESVTEQDSKDST

YSLSSTLTLSKADYEKHKVYACEVTHQGLSSPVTKSFNRGEC (SEQ ID NO: 119).

DIVLTQSPASLAVSLGQRATISCRASESVDSYGNSFMHWYQQKPGQPPKLLIYR
ASNLESGIPARFSGSGSRTDFTLTINPVEADDVATYYCQQSNEDPFTFGSGTKLEIKRTVAAPS
VFIFPPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC (SEQ ID NO: 120).

NIVMTQTPKFLLVSAGDRVTITRKASQSVSNDVVWYQQKPGQSPKLLMYYAS
YRYTGVPDRFTGSGYGTDFTFTISNVQAEDLAVYFCQQDYSSPFTFGSGTKLEIKRTVAAPS
VFIFPPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC (SEQ ID NO: 121).

DIVLTQSPASLAVSLGQRATISCRASQSVSTSTYNYMHWYQQKPGQPPKLLIKY
ASNLESGVPARFSGSGSGTDFTLNIHPVEEEDTATYYCQHSWEIPFTFGSGTKLEIKRTVAAP
SVFIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC (SEQ ID NO: 122).

The antibody composed of the SEQ ID NO: 101 and 112 above-described was called 3-6A4, the antibody composed of the SEQ ID NO: 102 and 113 above-described was called 5-20E11-5F2, and the antibody composed of the above SEQ ID NO: 103 and 114 was called 3-5B2-2G4, the antibody composed of the above SEQ ID NO: 104 and 115 was called 3-5B2-7A4, and the antibody composed of the above SEQ ID NO: 105 and 116 was called 3-2B5 -3D2, the antibody composed of the above SEQ ID NO: 106 and SEQ ID NO: 117 was called 3-7C9-1A6, and the antibody composed of the above SEQ ID NO: 107 and SEQ ID NO: 118 was called 3-9E1 -3A2, the antibody composed of the above SEQ ID NO: 108 and SEQ ID NO: 119 was called 3-15D5-3A6, and the antibody composed of the above SEQ ID NO: 109 and SEQ ID NO: 120 was called 3-15D5 -6A1, the antibody composed of the above SEQ ID NO: 110 and SEQ ID NO: 121 was called 5-20E11-1A2, and the antibody composed of the above SEQ ID NO: 111 and SEQ ID NO: 122 was called 5-21G2 -1D3.

### Example 5 Identification of Antibody Subtypes

Monoclonal hybridoma cell lines corresponding to the preferred antibody clones were injected into Balb/C mice using conventional methods to produce and purify ascites antibodies. Purified antibodies were used for antibody subtype analysis, and the antibody subtype detection method was carried out using a mature kit (Proteintech, KMIM-2). The results are shown in Table 4 below.

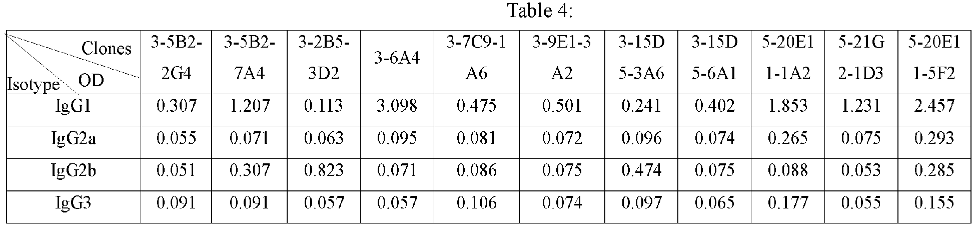

### Example 6 Expression and Purification of Monoclonal Antibodies

The present inventors constructed the preferred monoclonal antibody clones 3-6A4 and 5-20E11-5F2 sequences into conventional expression vectors using genetic engineering methods, and then expressed in CHO cells. After fermentation, the collected cell culture supernatants were purified by a Protein G chromatography column (EzFast Protein G 4FF, Borgron), the balance buffer was 20mM PBS, 0.15M NaCl, pH7.4, and the elution buffer was 0.1M glycine pH3.0±0.2, the protein eluate under the target absorption peak was collected, and after ultrafiltration with PBS buffer, and part of the samples were taken for SDS-PAGE electrophoresis detection, and the SEC-HPLC method was used to determine the aggregation content. The aggregation contents of the antibodies were all within 3%.

### Example 7 Antibodies Competitively Block the Binding of IL-11 to IL-11R

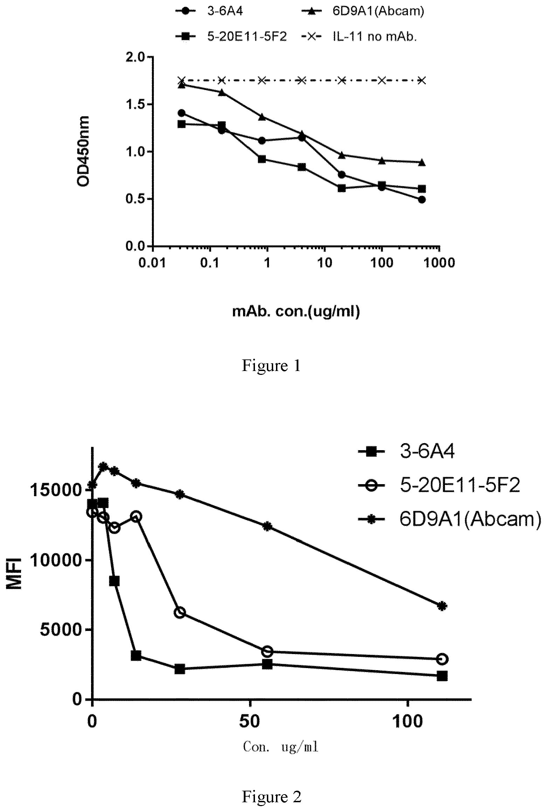

In order to detect the competitive binding situation of the preferred antibodies 3-6A4 and 5-20E11-5F2, the Elisa plates were coated with an appropriate amount of IL-11R-His protein and incubated overnight. Then, an appropriate amount of IL-11-Fc protein was added and co-incubated with different concentration gradients of antibodies. Finally, HRP-conjugated Goat Anti IgG-Fc secondary antibody (ThermoFisher, 31413) and TMB chromogenic substrate were used for detection. The inventors detected that both the preferred antibodies and 6D9A1 (Abcam, ab130083) could inhibit the binding of IL-11 to IL-11R to varying degrees. The results are shown in Figure 1.

### Example 8 Cross-Reactivity of Antibodies

IL-6 cytokine family members include IL-6, IL-11, CNTF, CLC, LIF, CT-1, OSM, IL-27, IL-31. IL-11 is a member of IL-6 cytokine family. The inventors used conventional Elisa method to coat recombinant IL-6 (Sinobiological-10395-HNAE), IL-27 (R&D systems, 2526-IL-010), LIF (Peprotech, 300-05), CLC (R&D systems, 962-CL-050), IL-11 (LSBio, LS-G132887-10), OSM (Genscript-z03132), IL-31 (Peprotech, 200-31), CNTF (R&D systems, 257-NT-010), CT- 1 (R&D systems, 612-CD-010) protein, and the binding affinity of the preferred antibodies 3-6A4 and 5-20E11-5F2 to members of the IL-6 cytokine family was detected. The results showed that the preferred antibodies do not cross-react with other members of the IL-6 cytokine family except for binding to IL-11 cytokine. The Elisa results (OD450 nm) are shown in Table 5 below.

**Table 5: Binding affinity detection of antibodies 3-6A4 and 5-20E11-5F2 with different members of the IL-6 cytokine family**

| | IL-6 | IL-27 | OSM | CLC | LIF | IL-31 | CNTF | CT-1 | IL-11 |
|---|---|---|---|---|---|---|---|---|---|
| 3-6A4 | 0.039 | 0.035 | 0.102 | 0.042 | 0.084 | 0.088 | 0.064 | 0.041 | 1.884 |
| 5-20E11-5F2 | 0.049 | 0.048 | 0.045 | 0.033 | 0.037 | 0.036 | 0.036 | 0.034 | 1.926 |

### Example 9 Binding Affinity Determination of IL-11 Monoclonal Antibodies to Antigen

Bio-Layer Interferometry (BLI) was used for measurement, and Ni-NTA sensor was used to capture the antigen IL-11 (His tag), the preferred antibodies 3-6A4 and 5-20E11-5F2 were selected as analytes. The analytes were diluted in 7 concentration gradients, the binding time was 60s and the dissociation time was 1000s. The buffer was 20mM PBS, 0.15M NaCl, 0.1% BSA, 0.05% Tween 20, pH7.4. BLI kinetics/affinity software was used to analysis and obtain the dissociation constant (kdis) and affinity constant (KD) of the preferred antibodies and the corresponding hu-IL-11 ligand. The specific results are shown in Table 6 below. In this example, Pall ForteBio (Octet QKe) instrument was used to determine the affinity of antibodies for antigens.

**Table 6:**

| Sample ID | Cone. (nM) | Response | KD (M) | kon(1/Ms) | kon Error | kdis(1/s) | kdis Error | Full X^2 | Full R^2 |
|---|---|---|---|---|---|---|---|---|---|
| 3-6A4 | 41.7 | 1.0442 | 3.93E-11 | 1.06E+06 | 2.08E+04 | 4.16E-05 | 2.39E-06 | 0.017992 | 0.999488 |
| 5-20E11-5F2 | 166.7 | 1.2727 | 6.90E-11 | 1.75E+05 | 3.19E+03 | 1.21E-05 | 2.94E-06 | 0.051363 | 0.999171 |

### Example 10 Competitive Blocking Activity Detection of Antibodies

In order to evaluate the binding of the preferred Anti-huIL-11 antibodies 3-6A4 and 5-20E11-SF2 to IL-11 and whether the binding between the ligand and the receptor can be blocked, the inventors constructed a cell line (membrane-IL-11R CHO) that stably expressing the full-length IL-11R, flow cytometry was used to detect the blocking activities of candidate antibodies and commercial antibody 6D9A1 (Abcam, ab130083) against IL-11 and IL-11R. Firstly, an appropriate amount of IL-11 recombinant protein with Fc tag was co-incubated with different gradients of antibodies for 15 mins, and then incubated with 1 E+05 membrane-II,-11R CHO cells. The secondary antibody (Goat-anti-Fc-FITC) (Abcam, ab98529) conjugated with FITC fluorophore was used for detection. The competitive binding results showed that the neutralizing activity IC50 of 3-6A4 antibody was about 7µg/mL; the neutralizing activity IC50 of the 5-20E11-5F2 antibody was approximately 21µg/mL, and the blocking activity of the candidate antibody was significantly better than that of the 6D9A1 clone antibody, the results are shown in Figure 2.

### Example 11 Antibodies Inhibit the Transformation of Lung Fibroblast HFL-1 into Fibrosis

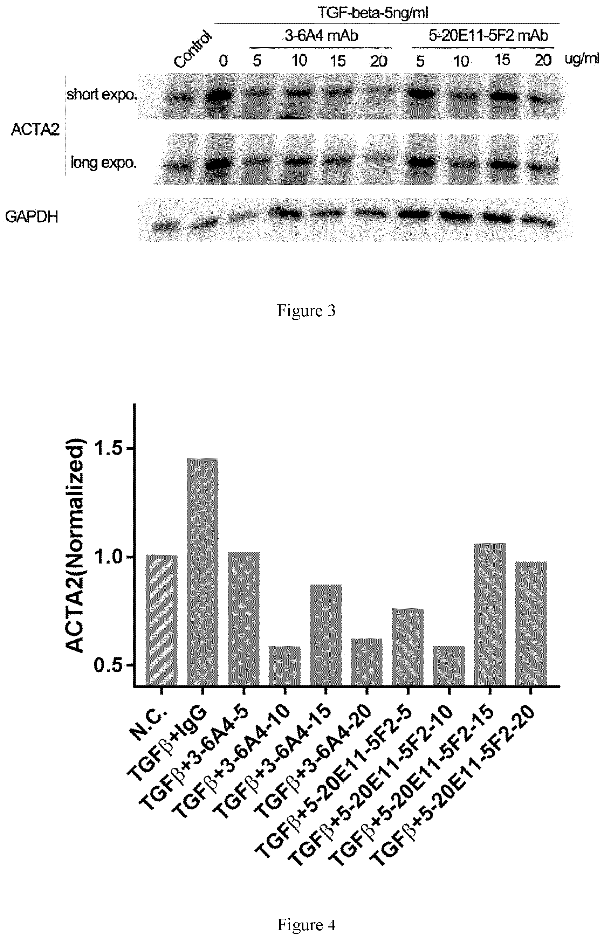

As lung fibroblasts, HFL-1 cells were induced by profibrotic factors such as TGF-beta1 (TGFβ) and expressed a large amount of IL-11 cytokines, and eventually transformed into fibrosis. When HFL-1 cells were transformed into fibrosis, the marker ACTA2 protein was accumulated continuously. In this example, the inventors used TGF-beta1 cytokine to induce HFL-1 cells to transform into fibrosis, and the preferred 3-6-A4 and 5-20E11-5F2 antibodies were added at the same time. After culturing for 48 hours, the cells were harvested for western detection and analysis. It can be seen that the preferred antibodies at different concentrations can inhibit the transformation of HFL-1 cells into fibrosis (the expression of ACTA2 protein was reduced) compared with the group that also added IgG (Mouse anti IgG control, Invitrogen, Catalog# 02-6502), as shown in Figure 3 and the corresponding statistical analysis Figure 4.

### Example 12 Antibodies Inhibit Pulmonary Fibrosis in C57BL/6 Mice

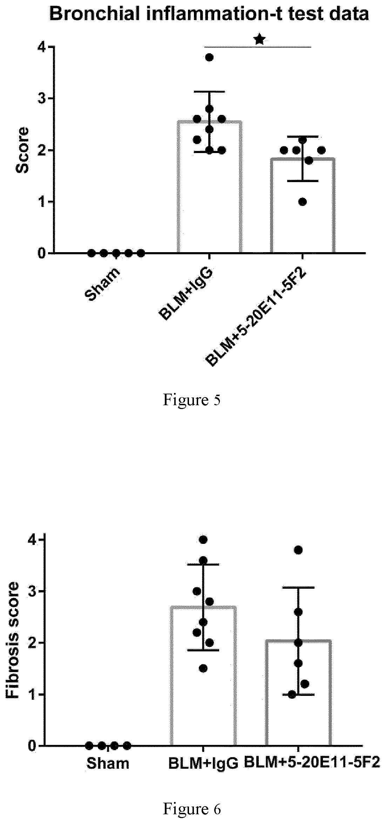

In the animal model of pulmonary fibrosis induced by bleomycin in C57BL/6 mice, specifically, 10-week-old C57BL/6 was subjected to general anesthesia with pentobarbital anesthesia, and the mainly trachea of the mouse lung was surgically exposed. Then an appropriate amount of bleomycin was injected into the trachea by instillation or nebulization. After seven days of bleomycin induction in mice, pulmonary fibrosis was basically appeared. On the eighth day, the inventors injected the preferred antibody 5-20E11-5F2 or the isotype control antibody IgG every other day. After 14 days of continuous injection, the mice were sacrificed and their lungs were harvested, and the inflammatory damage and fibrosis scores of the lungs were detected. Under this pharmacodynamic model, it can be seen that the candidate antibody can slow down pulmonary fibrosis in mice. The results are shown in Figs. 5 and 6.

### Example 13 Humanization of IL-11 Antibody

Complementarity determining region grafting (CDR grafting) was used to humanize the IL-11 antibody in the above embodiment. Firstly, the sequence of the human germline antibody with the highest homology to the light and heavy chain variable regions of the murine antibody was found, the germline selected for humanization of the antibody heavy chain variable region was IGHV1-46*01, IGKV3-11*01 was selected for humanization of the light chain variable region. The CDR region of the murine antibody was retained, and the framework sequence of the murine antibody was replaced with the framework sequence of the human germline antibody. Secondly, a structural model of the murine antibody Fv fragment was established, BLAST was performed in the PDB antibody database, and the structure INQB was selected as a template for homology modeling. Each different amino acid position in the structural model of the human antibody and the corresponding murine antibody was compared one by one. The amino acids that did not meet the FR structural specifications, the amino acids located in the FR core, and the amino acids located at the VH-VL interface were selected, and backmutated into murine sequence.

The sequences of the obtained heavy chain variable region and the light chain variable region of the IL-11 humanized antibody are shown in SEQ ID NO: 75 ~ 78 and SEQ ID NO: 79 ~ 82.

Sequence of IL-11 humanized antibody heavy chain variable region (VH1):

QVQLVQSGAEVKKPGASVKVSCKASDYTFTNYWLGWVRQAPGQGLEWMGHIYPGG
GYTNYNEKFKGRVTMTRDTSTSTVYMELSSLRSEDTAVYYCARVRSGNDALDFWGQGTLV
TVSS (SEQ ID NO: 75).

Sequence of II,-11 humanized antibody heavy chain variable region (VH2):

QVQLVQSGAEVKKPGASVKVSCKASDYTFTNYWLGWVRQAPGQGLEWIGHIYPGGG
YTNYNEKFKGRVTMTADTSTSTVYMELSSLRSEDTAVYFCARVRSGNDALDFWGQGTLVT
VSS (SEQ ID NO: 76).

Sequence of II,-11 humanized antibody heavy chain variable region (VH3):

QVQLVQSGAEVKKPGASVKVSCKASDYTFTNYWLGWVRQAPGQGLEWIGHIYPGG
GYTNYNEKFKGRATLTADTSTSTVYMELSSLRSEDTAVYFCARVRSGNDALDFWGQGTLVT
VSS (SEQ ID NO: 77).

Sequence of II,-11 humanized antibody heavy chain variable region (VH4):

QVQLVQSGAEVKKPGASVKVSCKASDYTFTNYWLGWVRQAPGHGLEWIGHIYPGG
GYTNYNEKFKGRATLTADTSTSTVYMELSSLRSEDTAVYFCARVRSGNDALDFWGQGTLVT

VSS (SEQ ID NO: 78).

Sequence of IL-11 humanized antibody light chain variable region (VL1):

EIVLTQSPATLSLSPGERATLSCRASESVDEYGISFMNWYQQKPGQAPRLLIYSASNQGS
GIPARFSGSGSGTDFTLTISSLEPEDFAVYYCQQSKEVPWTFGGGTKLEIK (SEQ ID NO: 79).

Sequence of IL-11 humanized antibody light chain variable region (VL2):

EIVLTQSPATLSLSPGERATLSCRASESVDEYGISFMNWYQQKPGQAPRLLIYSASNQGS
GIPARFSGSGSGTDFTLTISSLEPEDFAVYFCQQSKEVPWTFGGGTKLEIK (SEQ ID NO: 80).

Sequence of IL-11 humanized antibody light chain variable region (VL3):

EIVLTQSPATLSLSPGERATLSCRASESVDEYGISFMNWFQQKPGQAPRLLIYSASNQGS
GIPARFSGSGSGTDFTLTISSLEPEDFAVYFCQQSKEVPWTFGGGTKLEIK (SEQ ID NO: 81).

Sequence of IL-11 humanized antibody light chain variable region (VL4):

EIVLTQSPATLSLSPGERATLSCRASESVDEYGISFMNWFQQKPGQAPRLLIYSASNQGS
GIPARFSGSGSGTDFTLTISSLEPEDFAMYFCQQSKEVPWTFGGGTKLEIK (SEQ ID NO: 82).

The nucleic acid sequences encoding the light chain and heavy chain of the IL-11 humanized antibody were synthesized and inserted into the eukaryotic expression vector pcDNA3.4. The four heavy chain sequence vectors were paired with the four light chain sequence vectors respectively and co-transfected into mammalian cells CHO for expression to obtain single-chain antibody in the form of scFv. One week later, the supernatant containing scFv antibodies was collected for affinity detection.

### Example 14 Affinity Ranking of IL-11 Humanized Single Chain Antibody (scFv)

The binding of each humanized expression supernatant scFv obtained from Example 13 to IL-11 was determined by Biacore, wherein the connecting peptide in the humanized single-chain antibody was GGGGSGGGGSGGGGS (SEQ ID NO: 123), according to the dissociation constant kd (1/s), and three humanized antibodies with the smallest dissociation constant kd (1/s) were selected. The measure method included the steps of capturing the humanized single-chain antibody at a flow rate of 10 uL/min. Then the flow rate was switched to 30µL/min, and 400nM of IL-11 antigen was flowed through the sample channel, the binding time was 180s and the dissociation time was 600s. Finally, the chip was regenerated with 10mM glycine buffer. The results are shown in Table 7, and the humanized scFv "VH1+VL2" -scFv, "VH1+VL3" -scFv, and "VH1+VL4" -scFv dissociated from IL-11 more slowly than the other humanized scFv.

**Table 7: Affinity of IL-11 Humanized Antibodies to IL-11 Antigen**

| Ligand | Analyte | ka (1/Ms) | kd (1/s) | KD (M) | Rmax(RU) | Chi² (RU²) |
|---|---|---|---|---|---|---|
| BLANK(PBS) | IL-11 | NA | NA | NA | NA | NA |
| NC(culture medium) | IL-11 | NA | NA | NA | NA | NA |
| "VH1+VL1"-scFv | IL-11 | 3.51E+04 | 7.58E-04 | 2.16E-08 | 257.1 | 8.24E-01 |
| "VH1+VL2"-scFv | IL-11 | 4.13E+04 | 4.61E-04 | 1.12E-08 | 247.2 | 5.66E-01 |
| "VH1+VL3"-scFv | IL-11 | 3.55E+04 | 4.36E-04 | 1.23E-08 | 239.7 | 4.35E-01 |
| "VH1+VL4"-scFv | IL-11 | 3.31E+04 | 3.87E-04 | 1.17E-08 | 268.7 | 3.92E-01 |
| "VH2+VL1"-scFv | IL-11 | 5.34E+04 | 8.90E-04 | 1.67E-08 | 142.3 | 7.35E-01 |
| "VH2+VL2"-scFv | IL-11 | 4.57E+04 | 8.12E-04 | 1.78E-08 | 208.9 | 7.67E-01 |
| "VH2+VL3"-scFv | IL-11 | 4.56E+04 | 5.41E-04 | 1.19E-08 | 185.7 | 4.06E-01 |
| "VH2+VL4"-scFv | IL-11 | 4.88E+04 | 5.28E-04 | 1.08E-08 | 174.5 | 4.64E-01 |
| "VH3+VL1"-scFv | IL-11 | 4.45E+04 | 1.00E-03 | 2.25E-08 | 184.8 | 8.66E-01 |
| "VH3+VL2"-scFv | IL-11 | 4.65E+04 | 6.80E-04 | 1.46E-08 | 184.4 | 4.71E-01 |
| "VH3+VL3"-scFv | IL-11 | 4.76E+04 | 4.64E-04 | 9.76E-09 | 181.4 | 4.14E-01 |
| "VH3+VL4"-scFv | IL-11 | 4.18E+04 | 5.56E-04 | 1.33E-08 | 219.5 | 5.76E-01 |
| "VH4+VL1"-scFv | IL-11 | 4.89E+04 | 8.40E-04 | 1.72E-08 | 128.4 | 4.84E-01 |
| "VH4+VL2"-scFv | IL-11 | 5.52E+04 | 6.14E-04 | 1.11E-08 | 135.9 | 4.22E-01 |
| "VH4+VL3"-scFv | IL-11 | 4.61E+04 | 5.16E-04 | 1.12E-08 | 181.7 | 3.90E-01 |
| "VH4+VL4"-scFv | IL-11 | 4.79E+04 | 4.81E-04 | 1.00E-08 | 147.6 | 2.54E-01 |

### Example 15 Expression and Purification of Preferred IL-11 Humanized Antibodies

The preferred IL-11 humanized antibodies "VH1+VL2", "VH1+VL3", "VH1+VL4", and 5-20E11-5F2 (containing Fc fragments of IgG1) were respectively constructed with full-length sequences. The expression vectors pxC17.4 & pxC18.4 containing the full-length antibody were obtained and transfected into mammalian cells CHO for expression. After fermentation, the collected cell culture medium was purified by Protein A chromatography column. The balance buffer was 20mM PBS, pH7.4, and the eluent was 0.1M glycine pH3.0 ± 0.2. The protein eluent under the target absorption peak was collected. After ultrafiltration with PBS buffer, part of the samples was taken to measure the concentration by HPLC and purity by SDS-PAGE electrophoresis detection.

### Example 16 Affinity Detection of Preferred IL-11 Humanized Antibodies

The binding affinity of each preferred humanized full-length (IgG format) antibody sample to IL-11 obtained by Example 15 was measured using a surface plasmon resonance (SPR) biosensor Biacore T200 (GE Healthcare). The measure method included the following steps of capturing the full-length humanized antibody at a flow rate of 10µL/min. Then the flow rate was switched to 30µL/min, and different concentrations of IL-11 antigen (400nM, 200nM, 100nM, 50nM, 25nM, 12.5nM, 6.25nM) were passed through the sample channel and the reference channel. The binding time was 180s, and the dissociation time was 600s. Finally, the chip was regenerated with 10mM glycine buffer. As shown in Table 8, the affinity of the full-length humanized antibody constructed by "VH1+VL3" was better than that of the other two full-length humanized antibodies, and the affinity was slightly reduced compared with the full-length chimeric antibody constructed by 5-20E11-5F2.

**Table 8: Affinity of Preferred Humanized Antibodies to IL-11 Antigen**

| Antibody | Analyte | ka (1/Ms) | kd (1/s) | KD (M) | Rm ax (RU) | Chi² (RU²) |
|---|---|---|---|---|---|---|
| VH1+VL2 | IL-11 | 4.98E+04 | 5.97E-04 | 1.20E-08 | 114.1 | 4.03E-01 |
| VH1+VL3 | IL-11 | 5.26E+04 | 4.74E-04 | 9.03E-09 | 105.6 | 3.71E-01 |
| VH1+VL4 | IL-11 | 5.43E+04 | 5.45E-04 | 1.00E-08 | 63.26 | 1.25E-01 |
| 5-20E11-5F2 | IL-11 | 6.76E+04 | 4.33E-04 | 6.41E-09 | 72.58 | 6.99E-02 |

Among them, "VH1+VL2", "VH1+VL3" and "VH1+VL4" in Table 8 were all full-length (IgG form) humanized antibodies.

The heavy chain of the "VH1+VL2" full-length humanized antibody sequence was SEQ ID NO:85 and the light chain was SEQ ID NO: 90. The heavy chain of the "VH1+VL3" full-length humanized antibody sequence was SEQ ID NO: 85 and the light chain was SEQ ID NO: 91. The heavy chain of the "VH1+VL4" full-length humanized antibody sequence was SEQ ID NO: 85 and the light chain was SEQ ID NO: 92.

### Example 17 Thermal Stability Experiment of Preferred Humanized Antibodies

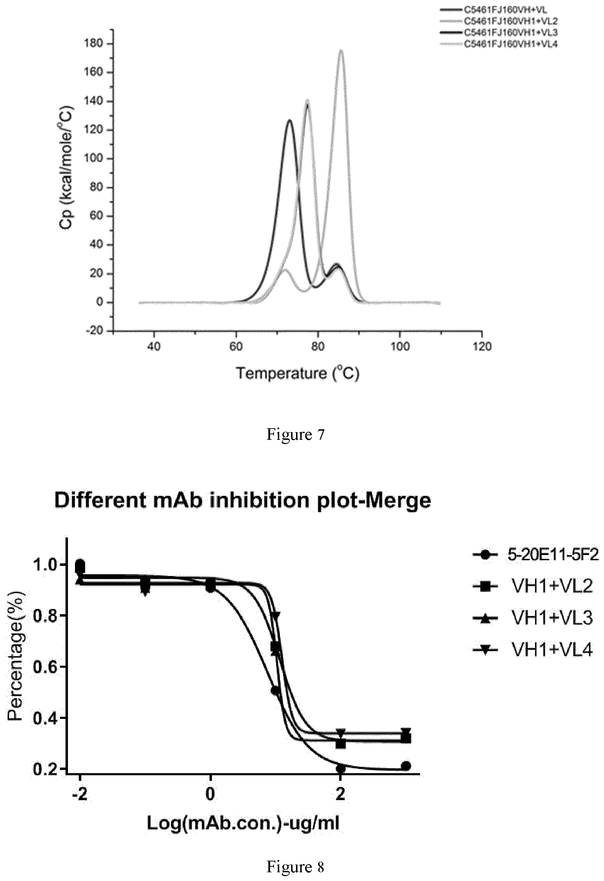

The thermal stability analysis of each full-length humanized or chimeric antibody obtained from Example 15 was determined by MicroCal^{™} VP-Capillary DSC system. The antibody was diluted to 0.4mg/mL with PBS buffer, and the assay conditions were as follows: heating rate was 90 °C /h, temperature was ranged 25 to 110 °C. The results are shown in Table 9 and Figure 7, the Tm value was increased 4.5 °C, compared the humanized antibody "VH1+VL3" with 5-20E11-5F2.

**Table 9: Tm values of preferred humanized antibodies**

| Antibody | Thalf | DH | TmOnset | Tm1 | Tm2 |
|---|---|---|---|---|---|
| 5-20E11-5F2 | 5.61 | 949000 | 63.38 | 72.99 | 84.46 |
| VH1+VL2 | 4.54 | 981000 | 65.51 | 71.91 | 85.52 |
| VH1+VL3 | 4.54 | 819000 | 67.6 | 77.49 | 85.23 |
| VH1+VL4 | 4.54 | 809000 | 68.25 | 77.34 | 85.08 |

Among them, "VH1+VL2", "VH1+VL3" and "VH1+VL4" in Table 9 and Figure 7 were all full-length (IgG form) humanized antibodies; "VH+VL" in Figure 7 was the 5-20E11-5F2 full-length antibody.

### Example 18 Competitively Blocking the Binding of IL-11 to IL-11R with Preferred Humanized Antibodies

To evaluate whether the preferred humanized antibody could block the binding of IL-11 to IL-11R. The inventors constructed a cell line (membrane-IL-11R CHO) that stably expressing IL-11R. The measure method included the following steps: 10 µL of II,-11-His protein at a concentration of 100 µg/mL and 90µL of serial dilutions of the full-length chimeric antibody 5-20E11-5F2 and full-length humanized antibodies constructed from" VH1+VL2 ", "VH1+VI,3" and "VH1+VL4" (100µg/mL, 10µg/mL, 1µg/mL, 0.1µg/mL, 0.01µg/mL, 0.001µg/mL) obtained from Example 15 were added to a 96-well U-shaped plate respectively and incubated for 1h at room temperature. Then 1.5*10^5 membrane-II,-11R CHO cells (100 µL/well) were added and incubated for 1h at room temperature. After incubation, the plate was centrifuged at 1500rpm for 5min, washed three times with PBS, and 1:400 dilution flow secondary antibody Goat-anti-Fc-FITC (Abcam, ab98529) was added with 200 µL/ well. Then, the plate was incubated at 4 °C for 1h, centrifuged at 1500rpm for 5min, washed three times with PBS, and resuspended cells in 180 µl PBS, and detected by flow cytometry. The results are plotted as S-curves in Figure 8, and the results show that the blocking activity of the humanized antibody was basically the same as that of the murine antibody.

**Table 10: EC50 of humanized antibody competitively blocking the binding of IL-11 to IL-11R**

| Antibody number | 5-20E11-5F2 | VH1+VL2 | VH1+VL3 | VH1+VL4 |
|---|---|---|---|---|
| EC50 (µg/mL) | 7.537 | 11.57 | 10.46 | 12.48 |

Among them, "VH1+VL2", "VH1+VL3" and "VH1+VL4" in Table 10 and Figure 8 were full-length (IgG form) humanized antibodies.

### Example 19 Preferred Humanized Antibodies Inhibit the Transformation of Hepatic Fibroblasts LX-2 to Fibrosis

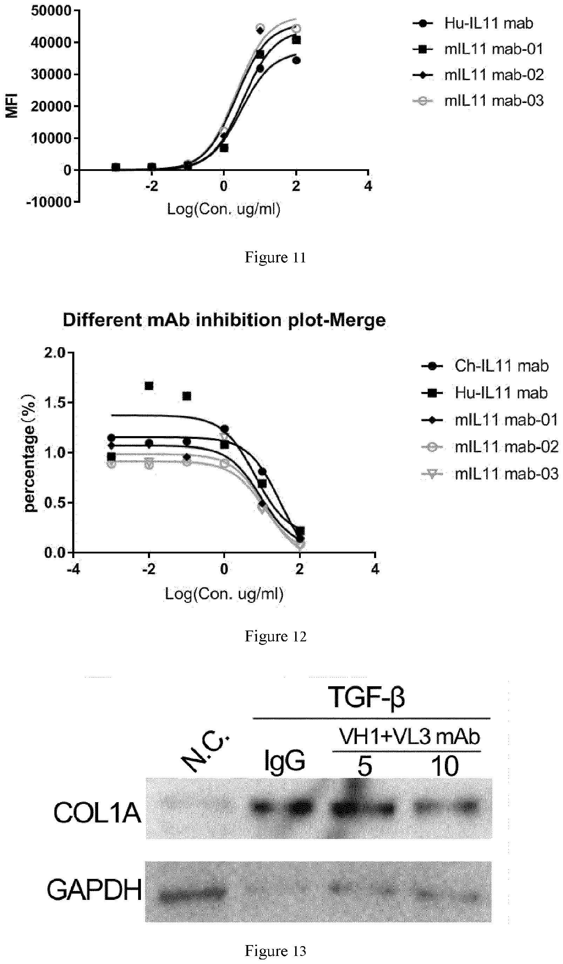

LX-2 cells are human hepatic stellate cells, which can express a large number of IL-11 cytokines when induced by pro-fibrotic factors such as TGF-beta1 (TGF-β), and eventually transform into fibrosis. The marker ACTA2 protein will accumulate during the LX-2 cells transform into fibrosis. In this example, LX-2 cells were plated in 6-well plates at a density of 3*10^5 cells/well and cultured for 10h until the cells adhered. After starvation in serum-free DMEM medium for 12h, 5ng/mL of TGF-beta1 cytokine was added for stimulation, and serial dilutions of the full-length humanized antibodies ("VH1+VL3" mAb 5µg/mL or 10µg/mL) constructed from "VH1+VL3" obtained form Example 15 were added at the same time. After culturing for 24h, the medium was taken, and 200µL RIPA lysate solution (Biyuntian, P0013B) was added to each well for western blot analysis. Results are shown in Figure 13, different concentrations of humanized antibodies can inhibit the transformation of LX-2 cells into fibrosis (the expression of Collagen IA protein was decreased), indicating that the preferred humanized antibody can well inhibit the transformation of LX-2 cells into fibrosis.

### Example 20 Preferred Antibodies Inhibit Pulmonary Fibrosis in SD Rats

In this example, the chemical drugs (HEC585 (ilfenidone hydrochloride), BIBF1120) and the full-length chimeric antibody constructed from 5-20E11-5F2 (CPD-1) and the full-length humanized antibody constructed from "VH1+VL3" (CPD-2, the "VH1+VL3" mAb) obtained from Example 15 were tested in animals. Among them, IgG antibody was used as a control. The specific experimental operations were as follows:

The rats were anesthetized with 2.5% isoflurane. After the main trachea was surgically opened, an appropriate amount of solvent or Bleomycin was injected to create a model. Among them, Bleomycin induction was used to construct an animal model of unilateral pulmonary fibrosis in SD rats, and the recovery of the rats was observed after the operation. On the 8th day after surgery, the animals were treated by gavage or intraperitoneal administration for 14 days. The specific animal groups and drug doses were summarized in Table 11 below. After the humanized antibody treatment, it was significantly observed that the humanized antibody slowed down the inflammatory response and fibrosis process in the lung of rats.

**Table 11: Rat efficacy experimental protocol of 5-20E11-5F2 and humanized antibodies (VH1+VL3)**

| Group | Number of animals | Modeling | Drug Information | Drug group | Dose of drug administered |
|---|---|---|---|---|---|
| G1 | 3 | No | - | Sham | N/A |
| G2 | 8 | Yes | - | IgG | 27mg/kg |
| G3 | 8 | Yes | Homemade | Yinfenidone | 5mg/kg |
| | | | | hydrochloride | |
| G4 | 8 | Yes | Nintedanib | BIBF1120 | 100mg/kg |
| G5 | 8 | Yes | 5-20E11-5F2 | CPD-1 | 9mg/kg |
| G6 | 8 | Yes | | CPD-1 | 27mg/kg |
| G7 | 8 | Yes | | CPD-1 | 54 mg/kg |
| G8 | 8 | Yes | VH1+VL3 | CPD-2 | 9mg/kg |
| G9 | 8 | Yes | | CPD-2 | 27mg/kg |
| G10 | 8 | Yes | | CPD-2 | 54 mg/kg |

Among them, "VH1+VL3" in Table 11 was a humanized full-length (IgG form) antibody.

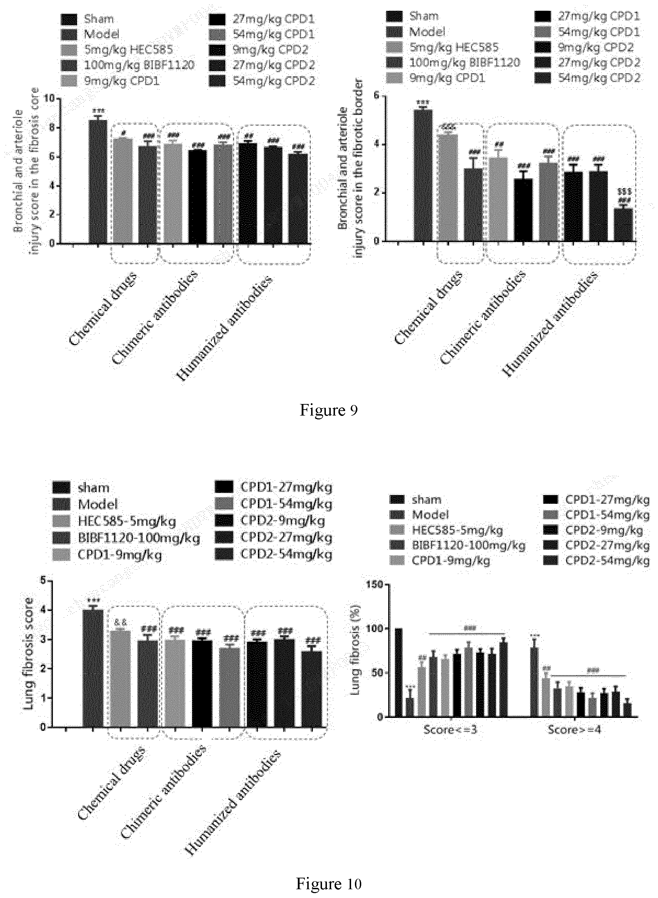

After the treatment, the lungs of the rats were taken for paraffin embedded and sliced up, and HE staining and Masson triple staining were performed. Among them, HE staining mainly indicates the infiltration level of inflammation in lung tissue. Masson's triple staining is mainly used to evaluate the level of pulmonary fibrosis. The results of Figures 9 and 10 show that the preferred humanized antibody can effectively reduce the inflammatory damage and fibrosis induced by Bleomycin in rats. In addition, the effect of humanized antibody on inflammatory damage in rats was significantly better than that of 5-20E11-5F2 antibody at high dose.

### Example 21 Affinity Maturation of Preferred Humanized Antibody

Antibody affinity maturation was performed using phage display technology. The ScFv in the form of VH-Linker-VL was constructed from the humanized antibody VH1+VL3 and used as a template for phage library construction. After several rounds of panning, the candidates with better affinity than the original sequence were selected by ELISA and flow cytometry. CDR mutation library and random mutation library were constructed, and the two libraries were constructed separately, and the phages were mixed during panning. (1) Primers were designed to construct four CDR mutation sublibraries, which were (VH-CDR3, VL-CDR3, VH-CDR1); (VH-CDR3, VL-CDR3, VH-CDR2); (VH-CDR3, VL-CDR3, VL-CDR1); (VH-CDR3, VL-CDR3, VL-CDR2), respectively, the four sublibraries were mixed in equal proportions before digestion. At the same time, a random mutation library was constructed, and ScFv sequences containing random mutation sites were obtained through multiple rounds of PCR by error-prone enzyme. These sequences were constructed into the phagemid vector as the random mutation library. The quality of the library and antibody diversity analysis were verified. (2) Antibody panning: different concentrations of IL-11 antigen were used in CDR mutation library and random mutation library to screen the phage that could bind IL-11 for several rounds of liquid phase screening, and the affinity characteristics were detected by phage ELISA and flow cytometry. Then 14 clones with good affinity activity were obtained by sequencing. The results of flow cytometry and sequencing are shown in Table 12. (3) Three preferred clones (1E12, 2E2, 1F12) were selected, the nucleic acid encoding the three clones and the IgG1 Fc fragment of wild type was used to construct full-length antibody PXC17.4 & PXC18.4 to express vectors.

**Table 12: Results of Affinity maturation sequencing**

| **Strain number** | **ELISA** | **Flow (Mean)** | **Amino acid mutation position** | **Amino acid mutations** |
|---|---|---|---|---|
| 1A10 | 2.058 | 2199 | 100,232 | VH-CDR3(R100S), VL-CDR3(V232Q) |
| 1B1 | 1.46 | 3501 | 10,72,87,158 | VH-FR1(E10V), VH-FR2(R72S, R87S), VL-CDR1(R158H) |
| 1E12 | 2.197 | 6317 | 100,108,189,192,227 | VH-CDR3(R100S, F108L), VL-CDR2(A189G,Q192E),VL-CDR3(Q227N) |
| 1F7 | 2.378 | 1859 | 35,155,183,192 | VH-CDR1(G35A), VL-FR1(L155V), VL-FR2(R183T), VL-CDR2(Q192L) |
| 1F9 | 2.589 | 3825 | 30,100,232 | VH-FR1(T30H), VH-CDR3(R100S), VL-CDR3(V232L) |
| 1F10 | 2.599 | 2637 | 108,194,231 | VH-CDR3(F108I), VL-CDR2(S194D), VL-CDR3(E231D) |
| 1F12 | 2.589 | 4141 | 100,193,232 | VH-CDR3(R100S), VL-CDR2(G193A), VL-CDR3(V232L) |
| 1G9 | 2.813 | 2245 | 13,131,192,214 | VH-FR1(K13Q), linking region (G131D), VL-CDR2(Q192R), VL-FR3(S214N) |
| 1G11 | 2.582 | 2897 | 100,193,232 | VH-CDR3(R100S), VL-CDR2(G193A), VL-CDR3(V232L) |
| 2E2 | 2.579 | 2098 | 100,232 | VH-CDR3(R100S), VL-CDR3(V232L) |
| 2E7 | 2.192 | 1988 | 54,100,232 | VH-CDR2(G54S), VH-CDR3(R100S), VL-CDR3(V232L) |
| 2F1 | 2.104 | 2388 | 100,232 | VH-CDR3(R100S), VL-CDR3(V232L) |
| 2F2 | 2.647 | 2004 | 100,108,122 | VH-CDR3(R100S, F108L), Linking region (G122E) |
| 2G9 | 2.086 | 1913 | 100,188,232 | VH-CDR3(R100S), VL-CDR2(S188G), VL-CDR3(V232L) |
| Original sequence | 1.156 | 450 | | |
| (1H9) | | | | |

### Example 22 Affinity Detection of Antibodies After Affinity Maturation

The binding of 1E12, 2E2, and 1F12 full-length antibody samples (numbered MIL-11 mab-01, MIL-11 mab-02, and MIL-11 mab-03, respectively) with mature affinity obtained from Example 21 to IL-11 was determined by Fortebio. The determination method consisted of the following steps: the humanized antibody was captured by an ARC sensor. The analytes were different concentrations of IL-11 antigen (125nM, 62.5nM, 31.3nM, 15.6nM, 7.81nM, 3.91nM), the binding time was 200s, the dissociation time was 1000 s. Finally, the sensor was regenerated with 10mM glycine (pH1.7) buffer. As shown in Table 13, the affinity of the affinity matured antibodies MIL-11 mab-02 and MIL-11 mab-03 increased 10-fold compared with the humanized antibody.

**Table 13: Affinity results of affinity maturation antibody**

| Ligand | Analyte | ka (1/Ms) | kd (1/s) | KD (M) |
|---|---|---|---|---|
| VH1+VL3 | IL-11 | 2.14E+05 | 6.09E-04 | 2.84E-09 |
| MIL-11 mab-01 | IL-11 | 5.01E+05 | 5.84E-04 | 1.17E-09 |
| MIL-11 mab-02 | IL-11 | 5.35E+05 | 1.30E-04 | 2.42E-10 |
| MIL-11 mab-03 | IL-11 | 5.80E+05 | 1.52E-04 | 2.62E-10 |

Among them, "VH1+VL3" in Table 13 was a humanized full-length (IgG form) antibody.

### Example 23 In Vitro Binding Activity Identification of affinity Maturation Antibodies by Flow Cytometry

The inventors constructed a membrane-IL-11 CHO cell line that stably expressed IL-11. Serial dilutions of affinity maturation antibodies (100µg/mL, 10µg/mL, 1µg/mL, 0.1µg/mL, 0.01µg/mL, 0.001µg/mL) obtained from Example 21 was incubated with 1.5*10^5 membrane-IL-11 CHO cells for 1h at room temperature. The cells were washed 3 times with PBS, and then the flow secondary antibody Goat-anti-Fc-FITC (Abcam, ab98529) was added and detected by flow cytometry. The results were plotted as S-curves in Figure 11. In the Figure, Hu-II,11 mab represents the humanized antibody constructed by "VH1+VL3". The results show that the binding activity of the affinity maturation antibody was basically the same as that of the humanized antibody.

### Example 24 In Vitro Blocking Activity Identification of affinity maturation antibodies by Flow Cytometry

To evaluate the difference between the preferred humanized antibody and the affinity maturation antibody in blocking the binding of IL-11 to IL-11R, a membrane-II,-11R CHO cell line that stably expressing IL-11R was constructed by the inventors. The measure method included the following steps: 10 µL of IL-11-Fc protein with a concentration of 10 µg/mL and 90 µL of serial dilutions of preferred humanized antibodies (100 µg/ml, 10 µg/ml, 1 µg/ml, 0.1 µg/ml, 0.01 µg/ml, 0.001 µg/ml) were taken and added to a 96-well U-shaped plate respectively and incubated for 1 h at room temperature. Then 1.5*10^5 membrane-IL-11R CHO cells (100 uL/well) were added and incubated for 1 h at room temperature. After incubation, the plate was centrifuged at 1500 rpm for 5 min, washed three times with PBS, and 1:200 diluted flow secondary antibody PE-anti-His Tag (Biogend, 362603) was added with 200 µl/well. Then, the plate was incubated at 4°C for 1 h, centrifuged at 1500 rpm for 5 min, washed three times with PBS, resuspended cells in 180 µL PBS, and detected by flow cytometry. The results were plotted as S-curves in Figure 12, wherein Ch-IL,11 mab represents the chimeric antibody constructed by "VH1+VL3". The results show that the blocking activity of the affinity maturation antibody was basically the same as that of the humanized antibody.

Reference throughout this specification to "an embodiment", "some embodiments", "one embodiment", "another example", "an example", "a specific example", or "some examples", means that a particular feature, structure, material, or characteristic described in connection with the embodiment or example is included in at least one embodiment or example of the present disclosure. Thus, the appearances of the phrases throughout this specification are not necessarily referring to the same embodiment or example of the present disclosure. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. An antibody or an antigen-binding fragment thereof that can specifically recognize IL-11, wherein the antibody comprises:
a heavy chain variable region comprising CDR1, CDR2 and CDR3 as shown in SEQ ID NO: 4, 5 and 6 or an amino acid sequence having at least 95% identity to SEQ ID NO: 4, 5 and 6, respectively; and
a light chain variable region comprising CDR1, CDR2 and CDR3 as shown in SEQ ID NO: 37, 38 and 39 or an amino acid sequence having at least 95% identity to SEQ ID NO: 37, 38 and 39, respectively; and
a heavy chain framework region comprising FL1, FL2, FL3 and FL4, and the FL1, FL2, FL3 and FL4 of the heavy chain framework region having an amino acid sequence shown in SEQ ID NO: 67, 68, 69 and 70, respectively,
QVQLVQSGAEVKKPGASVKVSCKAS (SEQ ID NO: 67),
LGWVRQAPGX1GLEWX2GH (SEQ ID NO: 68),
NYNEKFKGRX3TX4TXSDTSTSTVYMELSSLRSEDTAVYX6C (SEQ ID NO: 69),
WGQGTLVTVSS (SEQ ID NO: 70),
wherein, X1 is Q or H, X2 is M or I, X3 is V or A, X4 is M or L, X5 is R or A, and X6 is Y or F; and
a light chain framework region comprising FL1, FL2, FL3 and FL4, and the FL1, FL2, FL3 and FL4 of the light chain framework region having an amino acid sequence shown in SEQ ID NO: 71, 72, 73 and 74, respectively,
EIVLTQSPATLSLSPGERATLSCRAS (SEQ ID NO: 71),
MNWX7QQKPGQAPRLLIY (SEQ ID NO: 72),
NQGSGIPARFSGSGSGTDFTLTISSLEPEDFAX8YX9C (SEQ ID NO: 73),
FGGGTKLEIK (SEQ ID NO: 74),
wherein, X7 is Y or F, X8 is V or M, and X9 is Y or F.

2. The antibody or antigen-binding fragment thereof of claim 1, wherein the antibody has a heavy chain variable region with an amino acid sequence shown in any one of SEQ ID NO: 75 ~ 78.

3. The antibody or antigen-binding fragment thereof of claim 1, wherein the antibody has a light chain variable region with an amino acid sequence shown in any one of SEQ ID NO: 79 ~ 82.

4. The antibody or antigen-binding fragment thereof of claim 1, wherein the antibody comprises at least one of a heavy chain constant region and a light chain constant region, and at least a part of at least one of the heavy chain constant region and the light chain constant region is derived from at least one of a murine antibody, a human antibody, a primate antibody, or a mutant thereof.

5. The antibody or antigen-binding fragment thereof of claim 4, wherein the full-length sequence of the antibody constant region is shown in SEQ ID NO: 83 ~ 84.

6. The antibody or antigen-binding fragment thereof of claim 5, wherein the antibody has a heavy chain with an amino acid sequence shown in any one of SEQ ID NO: 85 ~ 88 and a light chain with an amino acid sequence shown in any one of SEQ ID NO: 89 ~ 92.

7. A nucleic acid molecule, wherein the nucleic acid molecule encodes the antibody or antigen-binding fragment thereof of any one of claims 1 ~ 6.

8. An expression vector, wherein the expression vector carries the nucleic acid molecule of claim 7.

9. A recombinant cell, wherein the recombinant cell carries the nucleic acid molecule of claim 7, or expresses the antibody or antigen-binding fragment thereof of any one of claims 1 ~ 6.

10. A pharmaceutical composition, wherein the pharmaceutical composition comprises the antibody of any one of claims 1 ~ 6, the nucleic acid molecule of claim 7, the expression vector of claim 8 or the recombinant cell of claim 9.

11. Use of the antibody of any one of claims 1 ~ 6, the nucleic acid molecule of claim 7, the expression vector of claim 8 or the recombinant cell of claim 9, the pharmaceutical composition of claim 10 in the manufacture of a medicament for treating or preventing pulmonary fibrosis, non-alcoholic steatohepatitis, chronic heart failure or diseases caused by the transformation of fibroblasts into fibrosis.

12. A kit for detecting IL-11, wherein the kit comprises the antibody of any one of claims 1 ~ 6.

13. Use of the antibody of any one of claims 1 ~ 6, the nucleic acid molecule of claim 7, the expression vector of claim 8, or the recombinant cell of claim 9 in the preparation of a kit for the detection of IL-11 or for the diagnosis of IL-11-related diseases.
